# EUROPEAN PATENT SPECIFICATION

(11) **EP 3 761 950 B1**
(45) Date of publication and mention of the grant of the patent: **09.04.2025**
(21) Application number: 19712012.4
(22) Date of filing: 28.02.2019
(51) Int. Cl.: A61Q 19/08, A61K 8/49, C07D 405/04, C07D 405/12, C07D 411/04, C07D 413/04, C07D 413/12

(54) **BIOISPIRED PROTEASOME ACTIVATORS WITH ANTIAGEING ACTIVITY**
BIOINSPIRIERTE PROTEASOM-AKTIVATOREN MIT ANTIAGING-AKTIVITÄT
ACTIVATEURS BIO-INSPIRÉS DES PROTÉASOMES AYANT UNE ACTIVITÉ ANTI-ÂGE

(30) Priority: 07.03.2018 GR 20180100094
(43) Date of publication of application: 13.01.2021
(73) Proprietor: Ioulia & Irene Tseti Pharmaceutical Laboratories Industrial and Commercial S.A., 14564 Kifissia (GR)
(72) Inventor: KOUFAKI, Maria, 11635 Athens (GR); CALOGEROPOULOU, Theodora, 11635 Athens (GR); CHONDROGIANNI, Niki, 11635 Athens (GR); PAPAHATJIS, Demetris, 11635 Athens (GR); GONOS, Efstathios, 11635 Athens (GR); FOTOPOULOU, Theano, 11635 Athens (GR); PROUSSIS, Kyriakos, 11635 Athens (GR); CHAZAPI, Evanthia, 11635 Athens (GR)
(74) Representative: Wibbelmann, Jobst
(86) International application number: PCT/GR2019/000018
(87) International publication number: WO 2019/171088

(56) References cited:
- EP-A2- 2 246 037
- MUNTEANU ADELINA ET AL: "Modulation of Proteasome Activity by Vitamin E in THP-1 Monocytes", IUBMB LIFE, vol. 59, no. 12, 1 January 2007 (2007-01-01), pages 771 - 780, XP055955088, ISSN: 1521-6543, DOI: 10.1080/15216540701697420
- KOUFAKI M ET AL: "Synthesis of a second generation chroman/catechol hybrids and evaluation of their activity in protecting neuronal cells from oxidative stress-induced cell death", BIOORGANIC & MEDICINAL CHEMISTRY, PERGAMON, GB, vol. 18, no. 11, 1 June 2010 (2010-06-01), pages 3898 - 3909, XP002637756, ISSN: 0968-0896, [retrieved on 20100421], DOI: 10.1016/J.BMC.2010.04.042
- PALOZZA P ET AL: "Design, synthesis, and antioxidant potency of novel @a-tocopherol analogues in isolated membranes and intact cells", FREE RADICAL BIOLOGY AND MEDICINE, ELSEVIER INC, US, vol. 44, no. 7, 1 April 2008 (2008-04-01), pages 1452 - 1464, XP022797594, ISSN: 0891-5849, [retrieved on 20080115], DOI: 10.1016/J.FREERADBIOMED.2008.01.001
- MARIA KOUFAKI ET AL: "Isoxazole substituted chromans against oxidative stress-induced neuronal damage", BIOORGANIC & MEDICINAL CHEMISTRY, PERGAMON, GB, vol. 19, no. 16, 26 June 2011 (2011-06-26), pages 4841 - 4850, XP028252903, ISSN: 0968-0896, [retrieved on 20110629], DOI: 10.1016/J.BMC.2011.06.074
- ANNIKA HÖHN ET AL: "Happily (n)ever after: Aging in the context of oxidative stress, proteostasis loss and cellular senescence", REDOX BIOLOGY : AN OFFICIAL JOURNAL OF THE SOCIETY FOR FREE RADICAL BIOLOGY AND MEDICINE, AN OFFICIAL JOURNAL OF THE SOCIETY FOR FREE RADICAL RESEARCH-EUROPE, AN AFFILIATE JOURNAL OF THE INTERNATIONAL SOCIETY FOR FREE RADICAL RESEARCH (SFRRI), vol. 11, 1 April 2017 (2017-04-01), NL, pages 482 - 501, XP055550469, ISSN: 2213-2317, DOI: 10.1016/j.redox.2016.12.001
- RICCARDO AMORATI ET AL: "Kinetic and Thermochemical Study of the Antioxidant Activity of Sulfur-Containing Analogues of Vitamin E", CHEMISTRY - A EUROPEAN JOURNAL, vol. 13, no. 29, 5 October 2007 (2007-10-05), DE, pages 8223 - 8230, XP055550452, ISSN: 0947-6539, DOI: 10.1002/chem.200700309
- X CHEN ET AL: "Multi-Target Compounds Acting in the Central Nervous System Designed From Natural Products", CURRENT MEDICINAL CHEMISTRY, 1 January 2013 (2013-01-01), pages 1673 - 1685, XP055552587, Retrieved from the Internet <URL:http://www.eurekaselect.com/108440/article> [retrieved on 20190206]

## Description

The present invention refers to hybrid compounds of hydroxytyrosol with chroman analogues and bioisosteres as proteasome activators, methods for their preparation as defined in the claims and their use in a method for treatment, prevention and mitigation/alleviation of symptoms of conditions related to reduced proteasome activitiy and function, including, not exlusively, ageing and age-related diseases as defined in the claims.

The invention pertains to the synthesis of structural proteasome activators (compounds that activate the multienzyme complex through direct interaction with it) as well as compounds that induce proteasome activation through transcriptional activation of the expression of its subunits. Moreover, based on the prior knowledge that proteasome activation correlates with anti-ageing activity on human fibroblasts, as well as at a multicellular, organismal level, the invention refers to the use of the compounds as anti-ageing agents. The anti-ageing properties result from the decrease of cellular oxidative stress through the reduction of the cellular levels of oxidized proteins, as well as through the activation and maintenance of the activity and function of the proteasome.

The proteasome constitutes the main cellular proteolytic mechanism and it is responsible of cellular detoxification from toxic agents, such as oxidized proteins that accumulate with age and accelerate the appearance of the ageing phenotype (N. Chondrogianni et al. Free Radic. Biol. Med. 2014, 71: 303-320). Previous studies in human primary fibroblasts have revealed that proteasome assembly and function are reduced during the progression of ageing, *in vitro* and *in vivo,* while the ageing phenotype may appear prematurely and irreversibly upon partial proteasome inhibition (N. Chondrogianni et al., 2000, Fibroblast cultures from healthy centenarians have an active proteasome. Exp. Gerontol. 2000, 35: 721-728, N. Chondrogianni et al., J. Biol. Chem 2003, 278: 28026-28037, N. Chondrogianni and E.S. Gonos, Biogerontology 2004, 5: 55-61).

It has been also shown that overexpression of proteasome in human primary fibroblasts leads to ameliorated response to oxidative stress while it promotes cellular lifespan prolongation, thus verifying that the proteasome represents a mechanism for cellular longevity (N. Chondrogianni et al., J. Biol. Chem. 2005, 280: 11840-11850). Moreover, it has been shown that healthy centanarians possess active proteasomes, regardless of their advanced age (N. Chondrogianni et al., Exp. Gerontol. 2000, 35: 721-728). More recent studies have revealed that genetic activation of the proteasome at a multi-cellular organismal level as well as proteasome activation through natural compounds confers similar positive results on the organismal lifespan. Proteasome activation constitutes a central mechanism for the decceleration of organsimal ageing and of the progression of age-related diseases such as Alzheimer's and Huntington's diseases, thus diseases that are related with accumulation of toxic proteins and their aggregates (N. Chondrogianni et al., FASEB J 2015, 29: 611-622, N. Papaevgeniou et al., Antiox. Redox Signal. 2016, 25: 855-869). The results so far suggest that activation of the proteasome complex constitutes a pioneer strategy for the decceleration of ageing and age-related diseases (N. Chondrogianni et al., Ageing Res. Rev. 2015, 23 (PtA): 37-55).

Research towards the development of small molecules as proteasome activators is scarce. Various classes of compounds, including denaturating agents (SDS), lipids and peptides, act as 20S proteasome activators at relatively high concentrations (Wilk and Chen, Mol Biol Rep. 1997, 24: 119-124). Betulinic acid, a pentacyclic triterpene, promotes proteasome activation through the selective enhancement of chymotrypsin-like proteasome activity without affecting the other two proteasome activities (Huang et al., FEBS Lett. 2007, 581: 4955-4959). Other studies have shown that carboranes may enhance all three peptidase activities of the 20S proteasome in the absence of the 11S complex (H. S. Ban et al. ChemMedChem, 2010, 5, 1236-1241). Recent studies refer to natural compounds, such as oleuropein, quercetin and 18α-glycyrrhetinic acid, as proteasome activators that deccelerate cellular ageing (M. Katsiki et al., Rejuvenation Res. 2007, 10: 157-172; Kapeta et al., J Biol Chem. 2010, 285: 8171-8184; N. Chondrogianni et al., Exp Gerontol. 2010, 45: 763-771; N. Papaevgeniou et al., Antiox. Redox Signal. 2016, 25: 855-869 and EP2246037). In another study, the modulation of proteasome activity by vitamin E in THP-1 monocytes was investigated (A. Munteanu et al., IUBMB Life. 2007, 59(12): 771-80.

Hydroxytyrosol, the main polyphenolic constituent of olive oil, is found in olive leaves and olives as metabolite of the proteasome activator oleuropein and shows a variety of biological properties, including antibacterial and antioxidant activity, protective activity against oxidative DNA damage, oxidation of LDL, peripheral neuropathy, as well as inhibition of platelet aggregation and inhibition of 5- and 12-lipoxygenase.

Moreover, compounds bearing the pharmacophore chroman ring of vitamin E, possess high cytoprotective activity against glutamate-induced neuronal damage (Koufaki M. et al. Bioorg. Med. Chem. 2011: 19, 4841-4850) as well as anti-oxidant and anti-ageing activity (Koufaki M. et al. Eur. J. Med. Chem. 2014, 83: 508-515).

Koufaki M. (Koufaki M. et al. Bioorg. Med. Chem. 2010: 18, 3898-3909) presents the synthesis of a second generation chroman/catechol hybrids and evaluation of their activity in protecting neuronal cells from oxidative stress-induced cell death.

Palozza P. (Palozza P. et al. Free Rad. Bio. Med. 2008: 44, 1452-1464) presents the synthesis, and antioxidant potency of novel α-tocopherol analogues in isolated membranes and intact cells.

Hohn A. (Hohn A. et al. Redox Biol. 2017: 11, 482-501) reviews the aging effects in the context of oxidative stress, proteostasis loss and cellular senescence.

Amorati R. (Amorati R. et al. Chem. Eur. J. 2007: 13, 8223-8230) relates to kinetic and thermochemical study of the antioxidant activity of sulfur-containing analogues of vitamin E.

Chn X. (Chen X. et al. Curr. Med. Chem. 2013: 20, 1673-1685) reviews multiple-target compounds acting in the central nervous system using natural products as lead resources.

The present invention refers to novel bioinspired hybrid compounds as defined in the claims, combining the structural features of hydroxytyrosol and the natural antioxidant vitamin E or its bioisostere in one scaffold, which act as proteasome activators and exhibit anti-ageing activity. In particular, the present invention includes hybrid compounds which act either as structural proteasome activators (activation through allosteric interaction) or as transcriptional activators. Hitherto, synthetic compounds acting as structural 20S proteasome activators have not been mentioned in the literature or in patents.

The compounds of the present invention as defined in the claims can be used in a method for treatment, prevention or mitigation/alleviation of the symptoms of conditions related to decreased proteasome function and activity, including, but not limited to exclusively, ageing and age-related diseases, or for the production of anti-ageing products, which due to their antioxidant properties and rejuvenating effects, could be used as cosmetics.

Commercially available anti-ageing cosmetics contain natural antioxidants (or esters thereof) or extracts. However, the majority of known natural antioxidants, exhibit problems of absorption or stability. Recent patents refer to the incorporation of hybrid antioxidant compounds (such as resveratrol/lipoic acid - Chanel-, ascorbic/lipoic acid) for the improvement of the stability of the antioxidants and increased absorption from the skin (US2009215881A1, WO2009014343A2). With regard to extracts, they are complex mixtures of compounds which may not all be beneficial to skin health. Additionally, some cosmetic preparations report proteasome activation (a quercetin ester, Korres) which is indirect (increased protein expression level, possibly via transcriptional activation leading to a quantitative increase in proteasome complex) and consequently possible side effects. Finally, the use of certain marine algae (Dior) has been reported that simply inhibit UV-mediated proteasome inactivation.

In particular, the present invention refers to hybrid compounds of formula I 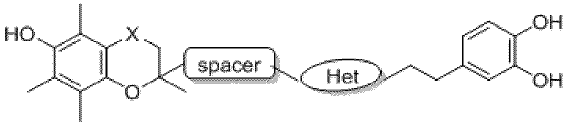 or optical isomers thereof wherein:
X = CH₂, S
Spacer = (CH₂)ₙ, n=0 or CH₂OCH₂, Het is selected from 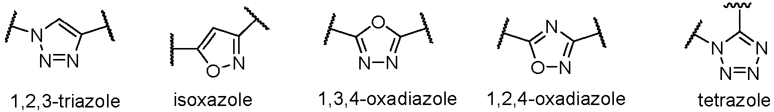 as defined in the claims.

Het is defined in the claims.

Generally, the compound can be in the form of pharmaceutically accepted esters of the compounds of formula I.

The present invention encompasses all possible stereoisomers of formula I as a mixture or as individual diastereomers insofrar as these are within the scope of the claims. If a compound of formula I is required as a single diastereomer or optical isomer, this can be obtained either by separation of the final diastereomers or optical isomers or by stereoselective synthesis using optically active compounds as starting materials or intermediates.

Generally, pharmaceutical formulations, methods, uses, crystalline forms (e.g. polymorphs), enantiomers, conformers of the compounds of formula I and pharmaceutically acceptable esters thereof can be provided. The compounds of the present invention may be labelled with isotopes, such as deuterium, tritium, carbon-11, carbon-14. The present invention encompasses all the isotopic variations of the described compounds.

The compounds of formula I activate the proteasome complex. These compounds can be used directly as pure substances or as part of a composition that increases proteasome activities.

The compounds of formula I are effective against fibroblasts replicative senescence (*in vitro* ageing), one of the major cell types of the epidermis that play an important role in tissue ageing.

The compounds of formula I are effective against the ageing of the multicellular nematode *C*. *elegans,* one of the basic organismal models of ageing.

The said compounds can act effectively against oxidative stress, causing a reduction in the intracellular levels of oxidized proteins in cultures of human fibroblasts undergoing replicative senescence.

The compounds of formula I and the pharmaceutically acceptable esters thereof may be used in a method for the treatment, prevention and mitigation/alleviation of symptoms of conditions associated with decreased proteasome activity, including, but not limited to, ageing and age-related diseases.

These compounds can be used in the production of anti-ageing preparations, pharmaceuticals and cosmetics.

The present invention also includes compositions containing at least one compound of formula I as defined in the claims as the active ingredient, optionally in combination with a pharmaceutically acceptable carrier or adjuvant.

The galenic formulations of the present invention can be administered in the known routes, including, but not limited to, topical, oral or transdermal administration. Additionally, the formulations of the present invention can be prepared for parenteral administration as injectable forms. The composition according to the invention can be prepared for slow release or for storage procedure. The mode of administration may be such that the active compound is transported to the appropriate site to exert its activity as a proteasome activator. Any other safe method of administration may be included in the above-mentioned administration methods.

Some compounds of formula I are listed as Examples below, the synthesis of which was carried out according to the Experimental Section shown below. These examples are given for a better understanding of the invention and are not meant to be limiting in any way.

The compounds of formula I of the present invention may be prepared as described in the following schemes and description, and relevant literature procedures may be used by those skilled in the art. The synthesis of the compounds of formula I, which are not specifically described, can be accomplished by methods analogous to those described in **Schemes 1-9** and Experimental Part as well as by related literature methods.

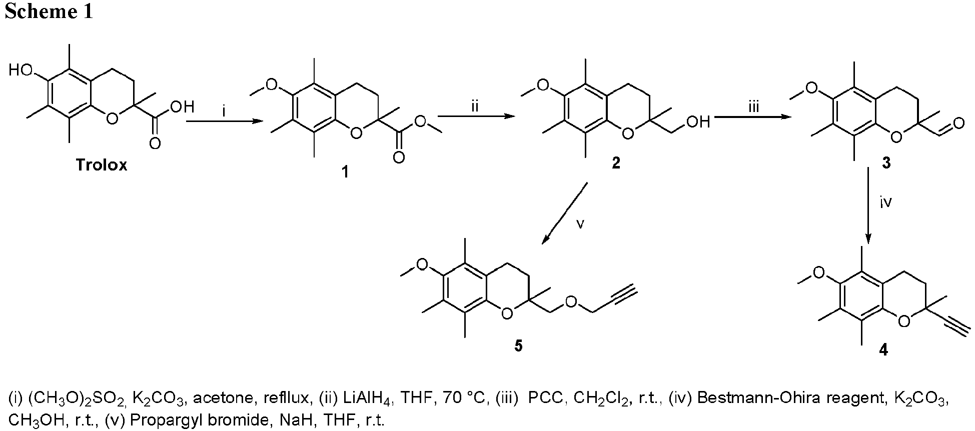

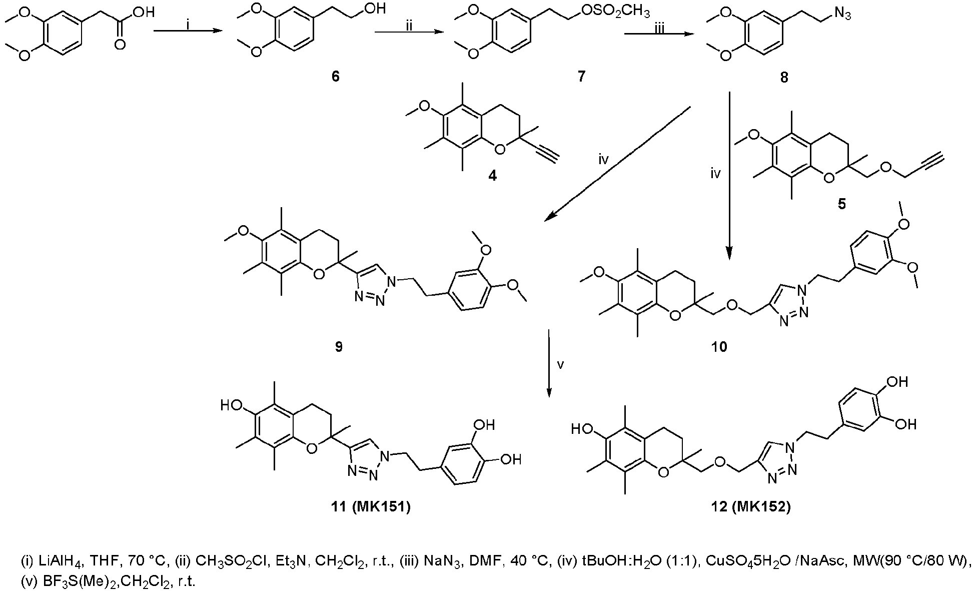

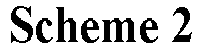

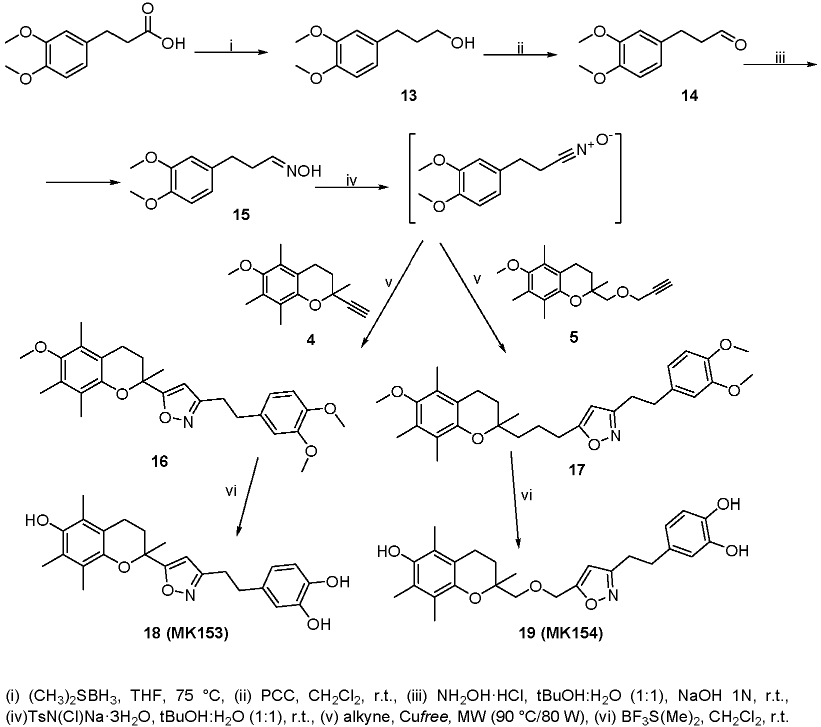

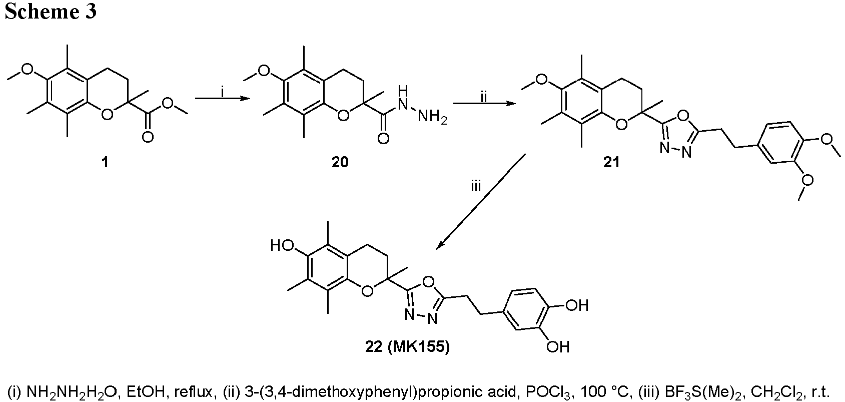

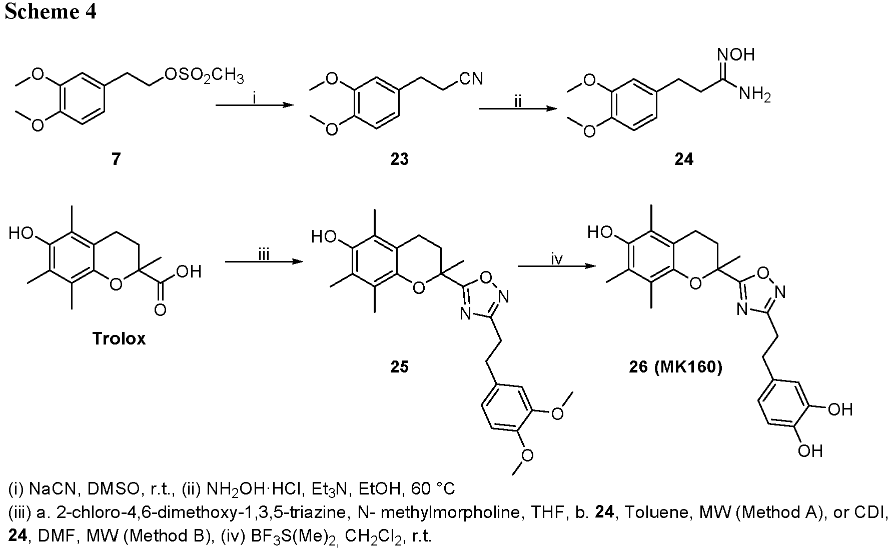

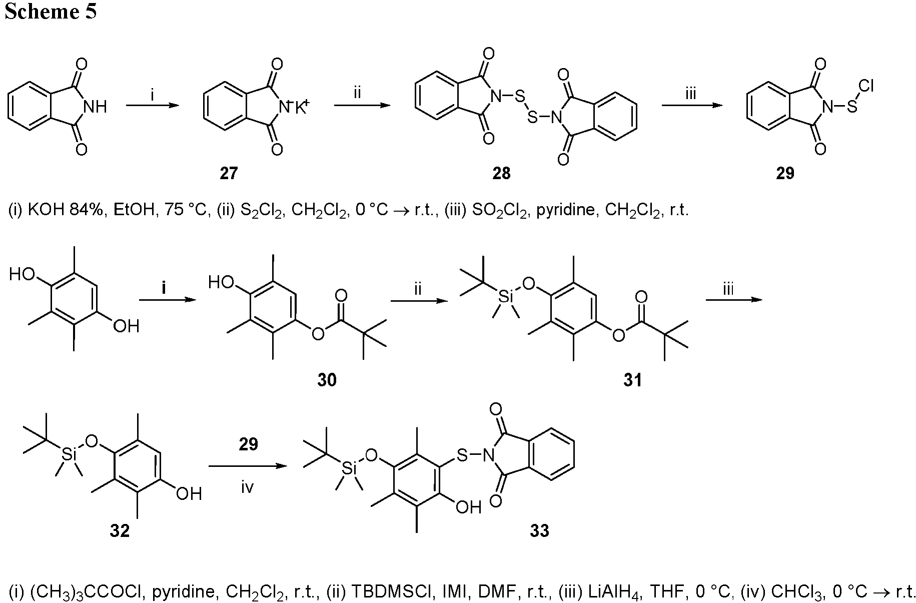

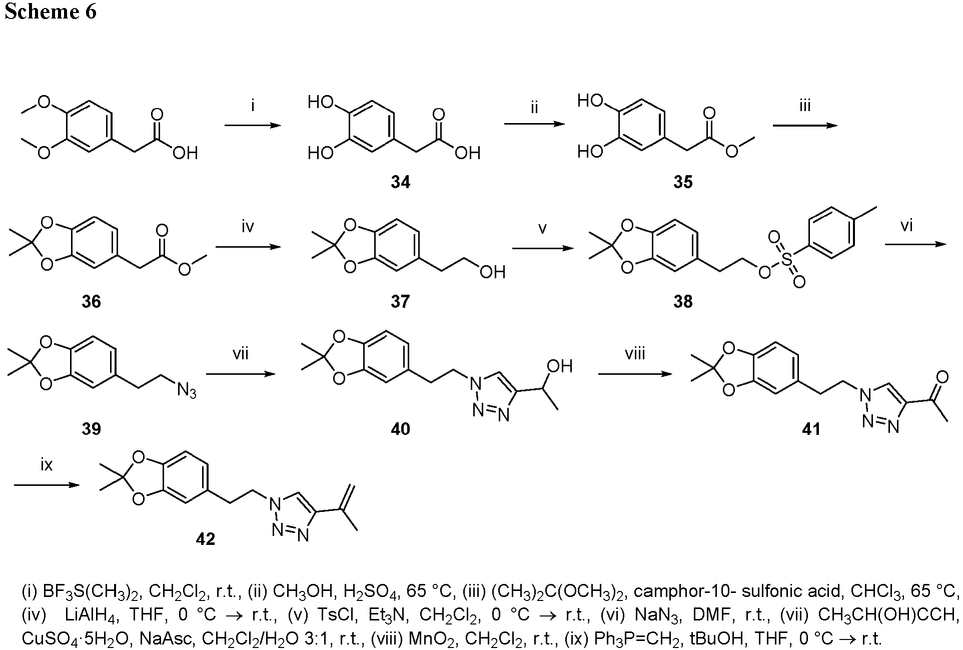

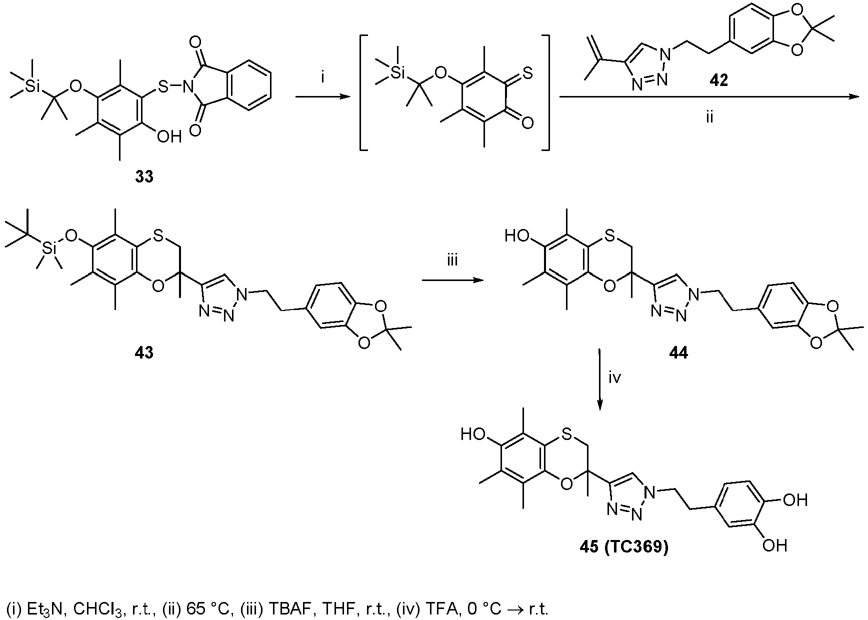

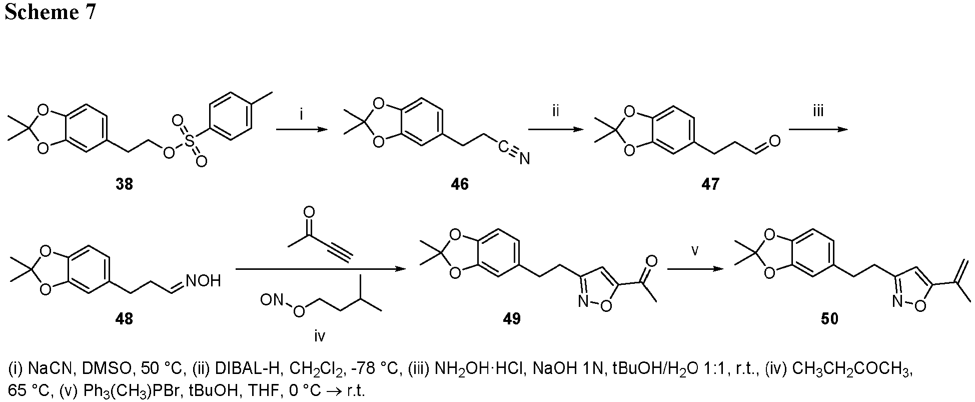

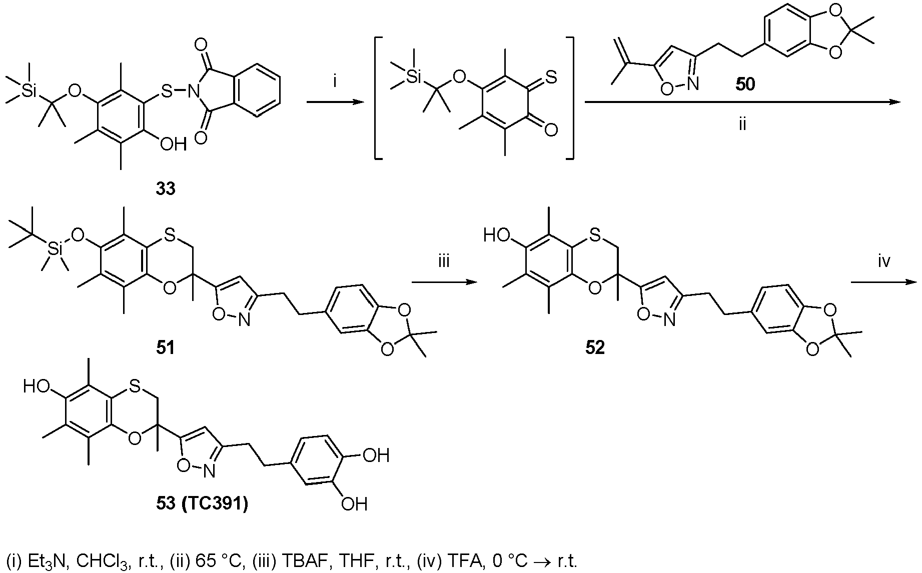

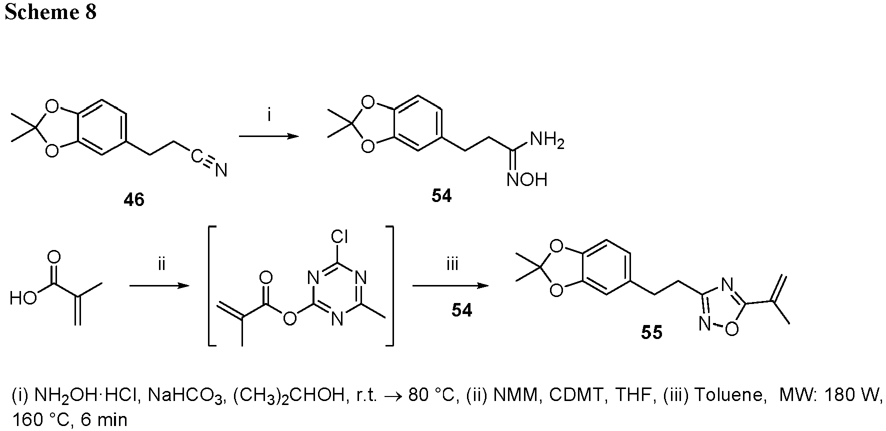

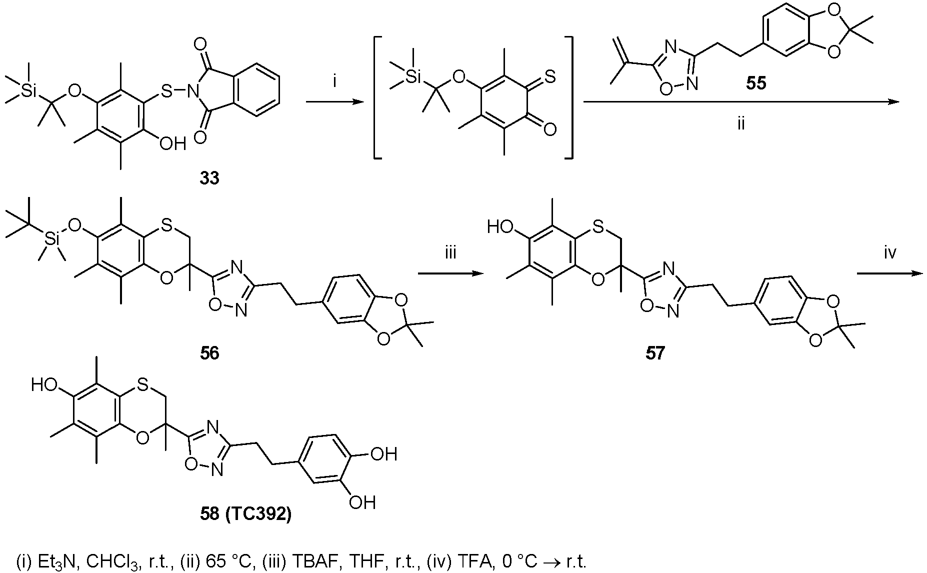

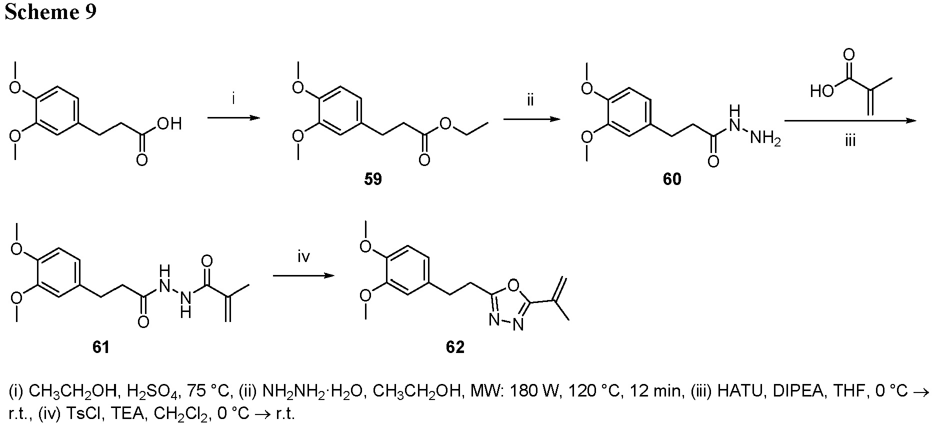

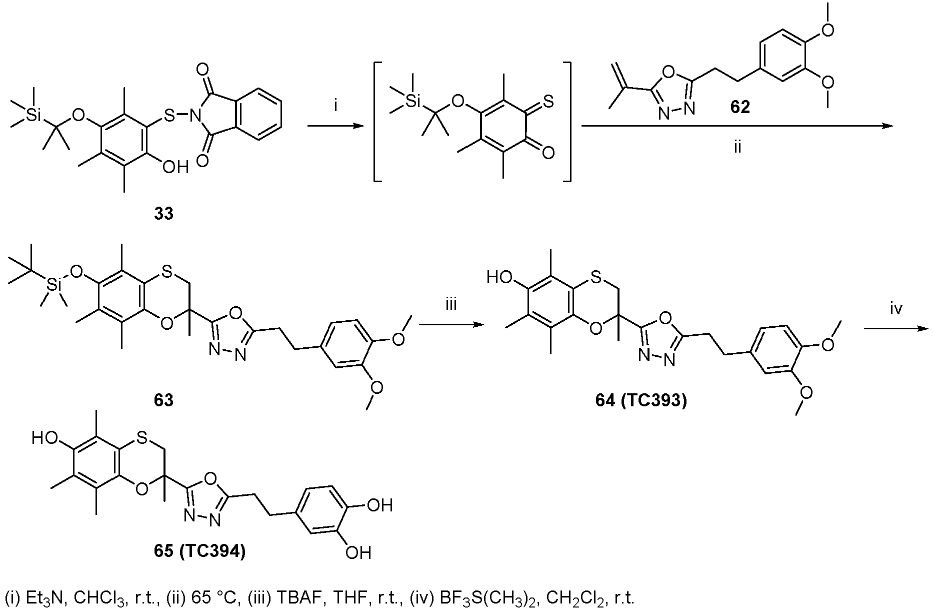

### EXPERIMENTAL PART

### SYNTHESIS OF NOVEL PROTEASOME ACTIVATORS

3,4-Dihydro-6-methoxy-2,5,7,8-tetramethyl-2H-1-benzopyran-2-carboxylic acid methyl ester **(1),** 3,4-dihydro-6-methoxy-2,5,7,8-tetramethyl-2H-benzopyran-2-methanol **(2),** 3,4-dihydro-6-methoxy-2,5,7,8-tetramethyl-2H-benzopyran-2-carboxaldehyde **(3),** 2-ethynyl-6-methoxy-2,5,7,8-tetramethyl-2H-1-benzopyran **(4)** and 3,4-dihydro-6-methoxy-2,5,7,8-tetramethyl-2-(prop-2-ynyloxymethyl)-2H-1-benzopyran **(5)** were prepared as shown in Scheme 1, according to the methods described in the following publications: Koufaki M., Theodorou E., Alexi X., Alexis M.N. Bioorg. Med. Chem. 2010, 18, 3898-3909. Koufaki M., Tsatsaroni A., Alexi X., Guerrand H., Zerva S., Alexis M.N. Bioorg. Med. Chem. 2011, 19, 4841-4850. Koufaki M., Fotopoulou T., Kapetanou M., Heropoulos G.A., Gonos E.S., Chondrogianni N. Eur. J. Med. Chem. 2014, 83, 508-515

**2-(3,4-Dimethoxyphenyl)ethan-1-ol (6)** To a suspension of LiAlH₄ (0.19 g, 5.07 mmol) in 3 mL dry THF, a solution of 2-(3,4-dimethoxyphenyl)acetic acid (0.50 g, 2.55 mmol) in 7 mL dry THF was added at 0 °C. The reaction mixture was gradually warmed to ambient temperature and then was refluxed for 30 min. A mixture of THF/H₂O (1:1) was added at 0 °C and then was diluted with ethyl acetate. Na₂SO₄ was added to the mixture and was stirred for 15 min. Finally, it was filtered through a pad of Celite and the filtrate was concentrated in vacuo giving the desired product as a white solid, (0.45 g, 98%). ¹H NMR (600 MHz, CDCl₃) *6:* 6.82-6.75 (m, 3H, ArH), 3.87 (s, 3H, -OC*H*₃), 3.85 (s, 3H, -OC*H*₃), 3.83 (t, *J*=6.5 Hz, 2H, -C*H*₂OH), 2.80 (t, *J*=6.5 Hz, 2H, -C*H*₂CH₂OH). ¹³C NMR (75 MHz, CDCl₃) *δ:* 148.5, 147.2, 130.9, 120.6, 112.0, 111.1, 63.3, 55.7, 55.4, 38.4. MS m/z: 182.82 (M+H)⁺, 204.96 (M+Na) ⁺, 386.64 (2M+Na)⁺

**3,4-Dimethoxyphenethyl methanesulfonate (7)** To a solution of 2-(3,4-dimethoxyphenyl)ethan-1-ol (6) in 10 mL dry CH₂Cl₂, Et₃N (0.4 mL) and CH₃SO₂Cl (0.2 mL, 2.42 mmol) were added at 0 °C and the reaction mixture was stirred at ambient temperature for 3 h. After completion of the reaction, water was added and then extracted with ethyl acetate. The organic layer was washed with saturated aqueous NaHCO₃, NaCl, dried over Na₂SO₄, filtered and the solvent was evaporated in vacuo. The desired product was obtained after purification by flash-column chromatography (PE/EtOAc, 60:40) as colorless oil, (0.46 g, 87%). ¹H NMR (600 MHz, CDCl₃) *δ:* 6.82-6.74 (m, 3H, ArH), 4.39 (t, *J*=7.0 Hz, 2H, -C*H*₂OSO₂CH₃), 3.87 (s, 3H, -OC*H*₃), 3.85 (s, 3H, -OC*H*₃), 2.99 (t, *J*=7.0 Hz, 2H, -C*H*₂CH₂OSO₂CH₃), 2.87 (s, 3H, - OSO₂C*H*₃). ¹³C NMR (75 MHz, CDCl₃) *δ:* 148.8, 147.9, 128.6, 120.9, 112.0, 111.2, 70.4, 55.8, 55.7, 37.2, 35.0. MS m/z: 282.88 (M+Na)⁺, 542.53 (2M+Na)⁺

**4-(2-Azidoethyl)-1,2-dimethoxybenzene (8)** To a solution of 3,4-dimethoxyphenethyl methanesulfonate (7) (0.12 g, 0.47 mmol) in 1.2 mL dry DMF, NaN₃ (0.04 g, 0.57 mmol) was added and the mixture was stirred at ambient temperature for 24 h. After completion of the reaction, the mixture was extracted with ethyl acetate. The organic layer was washed with saturated aqueous NaCl, dried over Na₂SO₄, filtered and the solvent was evaporated in vacuo. The desired product was obtained after purification by flash-column chromatography (PE/EtOAc, 90:10), as colorless oil, (0.09 g, 87%). ¹H NMR (300 MHz, CDCl₃) *δ:* 6.83-6.74 (m, 3H, Ar*H*), 3.88 (s, 3H, -OC*H*₃), 3.86 (s, 3H, -OC*H*₃), 3.48 (t, *J=7.2* Hz, 2H, -C*H*₂CH₂C-), 2.84 (t, *J=7.2* Hz, 2H, -CH₂C*H*₂N₃). ¹³C NMR (75 MHz, CDCl₃) *δ:* 148.9, 147.8, 130.5, 120.7, 111.8, 111.2, 55.8, 55.7, 52.6, 34.9. MS m/z: 229.98 (M+Na)⁺, 436.68 (2M+Na)⁺

**General procedure for the preparation of 1,4-disubstituted 1,2,3-triazoles.** To a solution of the appropriate alkyne (1eq) in a mixture of *t*-BuOH/H₂O (1:1), the azide (1.05 eq), CuSO₄·5H₂O (0.3 eq) and sodium ascorbate (0.6 eq), were added. The reaction was microwave irradiated (80 W, 90 °C, 30 min). After completion of the reaction, extraction with saturated aqueous NH₄OH and CH₂Cl₂ is performed. The organic layer was washed with NaCl, dried over Na₂SO₄, filtered and the solvent was evaporated in vacuo. The desired product was obtained after purification by flash-column chromatography (PE/EtOAc, 70:30). **1-(3,4-Dimethoxyphenethyl)-4-(3,4-dihydro-6-methoxy-2,5,7,8-tetramethyl-2*H*-benzopyran-2-yl)-1*H*-1,2,3-triazole (9)** According to the general procedure for the preparation of 1,4-disubstituted 1,2,3-triazoles, using **4** (0.02 g, 0.08 mmol) in 2.5 mL *t*-BuOH/H₂O (1:1) and 8 (0.02 g, 0.09 mmol), the desired product was obtained as colorless oil, (0.03 g, 95%). ¹H NMR (300 MHz, CDCl₃) *δ:* 6.96 (s, 1H, *H-*triazole), 6.74 (d, *J =* 7.8 Hz, 1H), 6.57 (d, *J = 7.9* Hz, 2H), 4.57 - 4.48 (m, 1H), 4.43 - 4.34 (m, 1H), 3.85 (s, 3H), 3.81 (s, 3H), 3.61 (s, 3H), 3.09 (t, *J =* 7.1 Hz, 2H), 2.64 - 2.47 (m, 2H), 2.35 - 2.25 (m, 1H), 2.19 (s, 3H, Ar-C*H*₃), 2.12 (s, 3H, Ar-C*H*₃), 2.09 - 2.02 (m, 4H, στo 2.06 (s, 3H, Ar-C*H*₃), 1.67 (s, 3H, -C*H*₃). ¹³C NMR (75 MHz, CDCl₃) *δ:* 152.8, 150.0, 149.1, 148.1, 147.4, 129.68, 128.0, 126.1, 122.5, 121.1, 120.8, 118.0, 111.8, 111.4, 74.0, 60.5, 56.0, 55.9, 51.9, 36.4, 32.1, 28.6, 20.8, 12.7, 11.9, 11.8. MS m/z: 452.09 (M+H)⁺, 474.24 (M+Na)⁺, 902.61 (2M)⁺, 924.52 (2M+Na)⁺. HRMS m/z: calcd for C₂₆H₃₄O₄N₃ [M+H]⁺ 452.25440, found for C₂₆H₃₄O₄N₃ 452.25450, calcd for C₂₆H₃₃O₄N₃Na [M+Na]⁺ 474.23631, found for C₂₆H₃₃O₄N₃Na 474.23631.

**1-(3,4-Dimethoxyphenethyl)-4-1[(3,4-dihydro-6-methoxy-2,5,7,8-tetramethyl-2*H*-benzopyran-2-yl)methoxy]methyl}-1*H*-1,2,3-triazole (10)** According to the general procedure for the preparation of 1,4-disubstituted 1,2,3-triazoles, using **5** (0.05 g, 0.18 mmol) in 2.5 mL *t*-BuOH/H₂O (1:1) and **8,** the desired product was obtained as colorless oil, (0.07 g, 84%). ¹H NMR (600 MHz, CDCl₃) *δ:* 7.23 (s, 1H, *H*-τρ 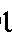 αζoλ 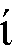 oυ), 6.77 (d, *J =* 8.1 Hz, 1H), 6.65 (dd, *J =* 8.1, 1.9 Hz, 1H), 6.55 (d, *J =* 1.8 Hz, 1H), 4.68 (ABq, 2H, Δν_{AB}=15.7 Hz, *J*_{AB}=12.5 Hz, -O-C*H*₂-), 4.53 (t, *J =* 7.2 Hz, 2H), 3.84 (s, 3H, -OC*H*₃), 3.81 (s, 3H, -OC*H*₃), 3.62 (s, 3H, -OC*H*₃), 3.50 (ABq, 2H, Δ*v*_{AB}=37.0 Hz, *J*_{AB}=9.9 Hz, -C*H*₂-O-), 3.14 (t, *J =* 7.2 Hz, 2H), 2.57 (t, *J =* 6.8 Hz, 2H), 2.17 (s, 3H, Ar-C*H*₃), 2.13 (s, 3H, Ar-C*H*₃), 2.07 (s, 3H, Ar-C*H*₃), 1.96 - 1.94 (m, 1H), 1.75 - 1.71 (m, 1H), 1.25 (s, 3H, -C*H*₃).¹³C NMR (75 MHz, CDCl₃) *6:* 149.8, 149.2, 148.2, 147.5, 129.6, 128.0, 125.9, 122.9, 120.8, 117.7, 111.9, 111.5, 75.9, 75.0, 65.4, 60.5, 56.0, 52.0, 36.5, 28.6, 22.3, 20.3, 12.7, 12.0, 11.8. MS m/z: 496.15 (M+H)⁺, 518.36 (M+Na)⁺, 990.65 (2M)⁺, 1012.82 (2M+Na)⁺. HRMS m/z: calcd for C₂₈H₃₈O₃N₃ [M+H]⁺ 496.28061, found for C₂₈H₃₈O₅N₃ 496.28097, calcd for C₂₈H₃₇O₃N₃Na [M+Na]⁺ 518.26251, found for C₂₈H₃₇O₅N₃Na 518.26241.

**3-(3,4-Dimethoxyphenyl)propan-1-ol (13)** To a solution of 3-(3,4-dimethoxyphenyl)-propionic acid (0.50 g, 2.38 mmol) in 10 mL THF, (CH₃)₂SBH₃ (0.3 mL, 3.57 mmol) was added at 0 °C and the mixture was stirred at 75 °C for 1.5 h. After the completion of the reaction, excess of (CH₃)₂SBH₃ was quenched by addition of CH₃OH at 0 °C and then the solvent was evaporated in vacuo. The product was used in the following step without further purification (0.47 g, 100%). ¹H NMR (300 MHz, CDCl₃) *δ:* 6.80 - 6.71 (m, 3H, Ar-*H*), 3.85 (s, 3H, -OC*H*₃), 3.84 (s, 3H, -OC*H*₃), 3.66 (t, *J =* 6.4 Hz, 2H, -C*H*₂OH), 2.64 - 2.62 (m, 2H, -C*H*₂CH₂CH₂OH), 1.91-1.81 (m, 2H, -CH₂C*H*₂CH₂OH).¹³C NMR (75 MHz, CDCl₃) *δ*: 148.9, 147.2, 134.5, 120.2, 111.8, 111.3, 62.3, 56.0, 55.9, 34.5, 31.8. MS m/z: 196.91 (M+H)⁺, 218.99 (M+Na)⁺, 414.67 (2M+Na)⁺

**3-(3,4-Dimethoxyphenyl)propanal (14)** To a solution of PCC (0.43 g, 2.0 mmol) in dry CH₂Cl₂ (4 mL), a solution of 3-(3,4-dimethoxyphenyl)propan-1-ol (0.30 g, 1.54 mmol) in dry CH₂Cl₂ (6 mL), was added at 0 °C. The mixture was stirred at ambient temperature for 2 h. After the completion of the reaction, the mixture was filtered through a pad of celite using Et₂O. The filtrate was concentrated in vacuo and the desired product was afforded as colorless oil and used without further purification, (0.18 g, 60%). ¹HNMR (600 MHz, CDCl₃) *δ:* 9.76 (s, 1H, -CHO), 6.75 - 6.68 (m, 3H, Ar-H), 3.82 (s, 3H, -OC*H*₃), 3.80 (s, 3H, - OC*H*₃), 2.86 (t, *J =* 7.5 Hz, 2H, -C*H*₂CH₂CHO), 2.73 - 2.70 (m, 2H, -CH₂C*H*₂CHO).¹³C NMR (75 MHz, CDCl₃) *δ:* 201.6, 148.9, 147.5, 132.9, 120.1, 111.7, 111.4, 55.9, 55.8, 45.4, 27.7. MS m/z: 411.12 (2M+Na)⁺

**General procedure for the preparation of 3,5-disubstituted isoxazoles** To a solution of 3-(3,4-dimethoxyphenyl)propanal (14) (1 eq) in a mixture of *t*-BuOH/H₂O (1:1), NH₂OH·HCl (1.05 eq) and 1N NaOH (1.05 eq), were added. The reaction mixture was stirred at ambient temperature for 1.5 h. After completion of oxime formation, TsN(Cl)Na·3H₂O (1.05 eq) was added in small portions, followed by the appropriate alkyne (1.05 eq) and the reaction was microwave irradiated (80 W, 90 °C, 45 min). Then, it was extracted with saturated aqueous NH₄OH and ethyl acetate. The organic layer was washed with NaCl, dried over Na₂SO₄ and the solvent concentrated in vacuo. The desired product was afforded after purification by flash-column chromatography (PE/EtOAc, 85:15).

**5-(3,4-Dihydro-6-methoxy-2,5,7,8-tetramethyl-2*H*-benzopyran-2-yl)-3-(3,4-dimethoxyphenethyl)-isoxazole (16)** According to the general procedure for the preparation of 3,5-disubstituted isoxazoles, using **4** (0.08 g, 0.32 mmol) in a mixture of 2.5 mL *t*-BuOH/H₂O (1:1), the desired product was obtained as colorless oil (0.05 g, 35%). ¹H NMR (300 MHz, CDCl₃) *δ:* 6.77 - 6.66 (m, 3H), 5.75 (s, 1H, *H-*isoxazole), 3.85 (s, 3H), 3.84 (s, 3H), 3.62 (s, 3H), 2.87 (bs, 4H), 2.66 - 2.57 (m, 1H), 2.49 - 2.37 (m, 2H), 2.21 (s, 3H, Ar-C*H*₃), 2.16 (s, 3H, Ar-C*H*₃), 2.11 - 1.99 (m κα 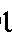 1s, 4H), 1.68 (s, 3H, -C*H*₃). ¹³C NMR (75 MHz, CDCl₃) *δ:* 175.2, 163.3, 150.3, 149.0, 147.6, 147.1, 133.4, 128.3, 126.0, 122.7, 120.3, 117.5, 111.8, 111.4, 100.8, 74.4, 60.5, 56.0, 55.9, 34.5, 31.3, 28.4, 27.3, 20.70, 12.7, 11.9, 11.8. MS m/z: 452.11 (M+H)⁺, 474.53 (M+Na)⁺. HRMS m/z: calcd for C₂₇H₃₄O₅N [M+H]⁺ 452.24310, found for C₂₇H₃₄O₅N 452.24339, calcd for C₂₇H₃₃O₅NNa [M+Na]⁺ 474.22511, found for C₂₇H₃₃O₅NNa 474.22542.

**5-{[(3,4-Dihydro-6-methoxy-2,5,7,8-tetramethyl-2*H*-1-benzopyran-2-yl)methoxy]-methyl}-3-(3,4-dimethoxyphenethyl)-isoxazole (17)** According to the general procedure for the preparation of 3,5-disubstituted isoxazoles, using **5** (0.08 g, 0.32 mmol), the desired product was obtained as colorless oil (0.04 g, 34%). ¹H NMR (600 MHz, CDCl₃) *δ:* 6.79 - 6.71 (m, 3H), 6.00 (s, 1H, *H*-isoxazole), 4.65 (ABq, 2H, Δ*v*_{AB}=18.8 Hz, *J*_{AB}=13.8 Hz, -O-C*H*₂-), 3.86 (s, 3H), 3.85 (s, 3H), 3.62 (s, 3H), 3.54 (ABq, 2H, Δv_{AB}=38.8 Hz, *J*_{AB}=10.0 Hz, -C*H*₂-O-), 2.99 - 2.91 (m, 4H), 2.59 (t,*J* = 6.5 Hz, 2H), 2.18 (s, 3H, Ar-C*H*₃), 2.14 (s, 3H, Ar-C*H*₃), 2.08 (s, 3H, Ar-C*H*₃), 2.00 - 1.95 (m, 1H), 1.78 - 1.73 (m, 1H), 1.28 (s, 3H, -C*H*₃). ¹³C NMR (75 MHz, CDCl₃) *δ:* 169.3, 163.3, 149.9, 149.0, 147.7, 147.4, 133.3, 128.1, 126.0, 122.9, 120.4, 117.6, 111.8, 111.4, 102.7, 76.4, 75.1, 64.8, 60.5, 56.0, 55.9, 34.2, 28.5, 28.3, 22.1, 20.3, 12.7, 12.0, 11.8. MS m/z: 518.30 (M+Na)⁺, 1012.38 (2M+Na)⁺. HRMS m/z: calcd for C₂₉H₃₈O₆N [M+H]⁺ 496.26941, found for C₂₉H₃₈O₆N 496.27018, calcd for C₂₉H₃₇O₆NNa [M+Na]⁺ 518.26130, found for C₂₉H₃₇O₆NNa 518.25200.

**3,4-Dihydro-6-methoxy-2,5,7,8-tetramethyl-2H-1-benzopyran-2-carbohydrazide (20)** It was prepared according to the method described in Koufaki M., Theodorou E., Alexi X., Alexis M.N. Bioorg. Med. Chem. 2010, 18, 3898-3909

**2-(3,4-Dimethoxyphenethyl)-5-(3,4-dihydro-6-methoxy-2,5,7,8-tetramethyl-2*H*-benzopyran-2-yl)-1,3,4-oxadiazole (21)** To a mixture of 3-(3,4-dimethoxyphenyl)-propionic acid (0.13 g, 0.59 mmol) and **20** (0.11 g, 0.40 mmol), POCl₃ (0.2 mL) was added and the reaction was heated at 100 °C for 2 h. The reaction was quenched with cold water and extracted with CH₂Cl₂. The organic layer was washed with saturated aqueous NaCl, dried over Na₂SO₄, filtered and the solvent was evaporated in vacuo. Purification by flash-column chromatography (AcOEt/PE, 95:5), afforded the desired product as colorless waxy solid, (0.10 g, 56%). ¹H NMR (300 MHz, CDCl₃) *δ:* 6.70 - 6.67 (m, 2H), 6.55 (dd, *J =* 8.2, 1.9 Hz, 1H), 3.83 (s, 6H, -OC*H*₃), 3.60 (s, 1H, -OC*H*₃), 3.12 - 3.07 (m, 2H), 3.01 - 2.96 (m, 2H), 2.77 - 2.59 (m, 3H), 2.20 - 2.07 (3s, 9H, Ar-C*H*₃), 1.72 (s, 3H, -C*H*₃). ¹³C NMR (75 MHz, CDCl₃) *δ:* 168.4, 166.7, 150.6, 149.0, 147.8, 146.8, 132.0, 128.2, 126.1, 123.2, 120.2, 117.5, 111.5, 111.3, 72.3, 60.4, 56.0, 55.9, 32.2, 30.6, 27.7, 26.5, 20.5, 12.6, 11.9, 11.7. MS m/z: 475.21 (M+Na)⁺, 904.68 (2M)⁺, 926.92 (2M+Na)⁺. HRMS m/z: calcd for C₂₆H₃₃O₃N₂ [M+H]⁺ 453.23840, found for C₂₆H₃₃O₅N₂ 453.23843, calcd for C₂₆H₃₂O₅N₂Na [M+Na]⁺ 475.22031, found for C₂₆H₃₂O₅N₂Na 475.22053.

**3-(3,4-Dimethoxyphenyl)propanenitrile (23)** To a solution of 3,4-dimethoxyphenethyl methanesulfonate (0.26 g, 1.01 mmol) in 3 mL dry DMSO, NaCN (0.25 g, 5.07 mmol) was added and the mixture was stirred at ambient temperature for 24 h. The reaction was quenched with cold water and extracted with Et₂O. The organic layer was washed with saturated aqueous NaCl, dried over Na₂SO₄, filtered and the solvent was evaporated in vacuo. The desired product was used without further purification (0.15 g, 79%). ¹H NMR (600 MHz, CDCl₃) *δ:* 6.82-6.74 (m, 3H, ArH), 3.87 (s, 3H, -OC*H*₃), 3.86 (s, 3H, -OC*H*₃), 2.88 (t, *J*=7.3 Hz, 2H, -C*H*₂CH₂CN), 2.57 (t, *J*=7.3 Hz, 2H, -C*H*₂CN). ¹³C NMR (75 MHz, CDCl₃) *δ:* 149.0, 148.1, 130.5, 120.2, 119.2, 111.4, 111.3, 55.9, 55.8, 40.8, 31.1, 19.6. MS m/z: 213.99 (M+Na)⁺, 404.62 (2M+Na)⁺

**3-(3,4-Dimethoxyphenyl)-N'-hydroxypropanimidamide (24)** To a solution of 3-(3,4-dimethoxy phenyl)propanenitrile **(23)**(0.09 g, 0.47 mmol) in 5 mL EtOH, NH₂OH·HCl (0.15 g, 2.35 mmol) and Et₃N (0.3 mL, 2.35 mmol) were added and the mixture was stirred at 45 °C for 48 h. Solvent was evaporated and the residue was extracted with ethyl acetate. The organic layer was washed with saturated aqueous NaCl, dried over Na₂SO₄ and the solvent was evaporated in vacuo. The desired product was afforded after purification by flash-column chromatography (CH₂Cl₂/MeOH, 95:5), as white solid, (0.06 g, 57%). ¹H NMR (300 MHz, CDCl₃) *δ:* 6.80-6.73 (m, 3H, ArH), 4.57 (bs, 2H, -N*H*₂), 3.86 (s, 3H, -OC*H*₃), 3.84 (s, 3H, -OC*H*₃), 2.83 (t, *J*=7.4 Hz, 2H, -CH₂C*H*₂C-), 2.44 (t, *J*=7.4 Hz, 2H, -C*H*₂CH₂C-). ¹³C NMR (75 MHz, CDCl₃) *δ:* 153.7, 148.8, 147.4, 133.1, 120.0, 111.5, 111.2, 55.8, 55.7, 33.1, 32.6. MS m/z: 225.03 (M+H) ⁺, 448.69 (2M)⁺, 470.65 (2M+Na)⁺

### 2-(3-(3,4-Dimethoxyphenethyl)-1,2,4-oxadiazol-5-yl)-2,5,7,8-tetramethyl-2H-1-benzopyran-6-ol (25)

**Method A.** To a solution of trolox (0.04 g, 0.16 mmol) in 1.5 mL dry THF, 2-chloro-4,6-dimethoy-1,3,5-triazine (0.03 g, 0.17 mmol) and N-methylmorpholine (0.05 mL, 0.43 mmol) were added and the reaction mixture was stirred at ambient temperature for 1 h. Then, 3-(3,4-dimethoxyphenyl)-N'-hydroxypropanimidamide **(24)** (0.03 g, 0.17 mmol) and 1 mL toluene were added and the reaction was microwave irradiated (160 °C, 250 W, 7 min). After completion of the reaction, the mixture was extracted with ethyl acetate. The organic layer was washed with saturated aqueous NaCl, dried over Na₂SO₄ and the solvent was evaporated in vacuo. The desired product was afforded after purification by flash-column chromatography (PE/AcOEt, 70:30) (0.04 g, 60%).

**Method B.** To a suspension of CDI (0.05 g, 0.33 mmol) in 3 mL dry DMF, trolox (0.07 g, 0.30 mmol) was added and the reaction mixture was stirred at ambient temperature for 3 h. Then, 3-(3,4-dimethoxyphenyl)-N'-hydroxypropanimidamide **(24)** (0.03 g, 0.17 mmol) was added and stirring was continued at the same temperature for 1 h. Afterwards, the reaction was microwave irradiated (150 °C, 180 W, 15 min). After completion of the reaction, it was extracted with Et₂O. The organic layer was washed with saturated aqueous NaCl, dried over Na₂SO₄ and the solvent was evaporated in vacuo. The desired product was afforded after purification by flash-column chromatography (PE/AcOEt, 60:40), (0.06 g, 50%). ¹H NMR (600 MHz, CDCl₃) *δ:* 6.72 (d, *J =* 8.2 Hz, 1H), 6.70 (d, *J =* 1.9 Hz, 1H), 6.64 (dd, *J =* 8.1, 1.9 Hz, 1H), 3.84 (s, 3H), 3.82 (s, 3H), 2.98 (s, 4H), 2.70 - 2.61 (m, 3H), 2.21 (s, 3H, Ar-C*H*₃), 2.16 (s, 3H, Ar-C*H*₃), 2.16 - 2.11 (m, 1H), 2.05 (s, 3H, Ar-C*H*₃), 1.76 (s, 3H, -C*H*₃). ¹³C NMR (75 MHz, CDCl₃) *δ:* 181.4, 170.0, 149.2, 147.8, 146.0, 145.1, 133.1, 123.3, 121.7, 120.5, 118.7, 117.0, 111.9, 111.5, 73.9, 56.2, 56.1, 32.9, 31.7, 28.5, 27.2, 20.8, 12.5, 12.2, 11.5. MS m/z: 439.29 (M+H)⁺, 461.34 (M+Na)⁺, 899.71 (2M+Na)⁺. HRMS m/z: calcd for C₂₅H₃₁O₅N₂ [M+H]⁺ 439.22270, found for C₂₅H₃₁O₅N₂ 439.22187, calcd for C₂₅H₃₀O₅N₂Na [M+Na]⁺ 461.20470, found for C₂₅H₃₀O₅N₂Na 461.20363.

**General method for the preparation of the final hydroxy-analogues.**To a solution of the appropriate protected compound (1 mmol) in dry CH₂Cl₂ (0.05 M), BF₃S(Me)₂ (10 equiv for each methoxy group) was added at 0 °C and the reaction mixture was stirred at ambient temperature for 24 h. After completion of the reaction, the solvent and excess reagent were evaporated under argon stream. The residue was extracted with ethyl acetate. The organic layer was washed with saturated aqueous NaCl, dried over Na₂SO₄ and the solvent was evaporated in vacuo. Purification by flash-column chromatography (CH₂Cl₂/MeOH, 95:5), afforded the desired product.

### Example 1

**1-(3,4-Dihydroxyphenethyl)-4-(3,4-dihydro-6-hydroxy-2,5,7,8-tetramethyl-2*H*-benzopyran-2-yl)-1*H*-1,2,3-triazole (11, MK151)** According to the general method for the preparation of the final hydroxy-analoques using the protected analoque **9** (0.06 g, 0.12 mmol), the desired product was obtained as white solid, (0.04 g, 82%). ¹H NMR (300 MHz, CDCl₃) *δ:* 6.95 (s, 1H, H-triazole), 6.83 (d, *J =* 1.7 Hz, 1H), 6.67 (d, *J =* 8.0 Hz, 1H), 6.37 (d, *J =* 1.9 Hz, 1H), 4.49 - 4.38 (m, 2H), 3.02 (t, *J =* 7.3 Hz, 2H), 2.57 - 2.51 (m, 2H), 2.18 - 2.02 (m, 11H, 2.18, 2.15 and 2.02 3s, Ar-C*H*₃), 1.66 (s, 3H, -C*H*₃ ). ¹³C NMR (150 MHz, CDCl₃) *δ:* 145.0, 144.8, 144.3, 143.1, 128.8, 121.9, 121.8, 121.6, 120.1, 119.2, 117.3, 115.0, 114.9, 73.4, 51.8, 35.8, 31.9, 28.7, 20.7, 12.2, 11.6, 11.2. MS m/z: 410.09 (M+H)⁺, 432.23 (M+Na)⁺, 818.54 (2M)⁺, 840.91 (2M+Na)⁺. HRMS m/z: calcd for C₂₃H₂₈O₄N₃ [M+H]⁺ 410.20740, found for C₂₃H₂₈O₄N₃ 410.20730, calcd for C₂₃H₂₇O₄N₃Na [M+Na]⁺ 432.18940, found for C₂₃H₂₇O₄N₃Na 432.18910, calcd for C₂₃H₂₆O₄N₃ [M-H]⁻ 408.19291, found for C₂₃H₂₆O₄N₃ 408.19195.

### Example 2

**1-(3,4-Dihydroxyphenethyl)-4-1[(3,4-dihydro-6-hydroxy-2,5,7,8-tetramethyl-2*H*-benzopyran-2-yl)methoxy]methyl}-1*H*-1,2,3-triazole (12, MK152)** According to the general method for the preparation of the final hydroxy-analoques using the methylated analoque **10** (0.07 g, 0.13 mmol), the desired product was obtained as white solid, (0.03 g, 58%). ¹H NMR (600 MHz, CDCl₃) *δ:* 7.23 (s, 1H, *H*-triazole), 6.74 (d, *J* = 8.0 Hz, 1H), 6.66 (d,*J* = 1.8 Hz, 1H), 6.50 (dd, *J* = 8.0, 1.8 Hz, 1H), 4.68 (ABq, 2H, Δ*ν*_{AB}=19.1 Hz, *J*_{AB}=12.6 Hz, -O-C*H*₂-), 4.51 (t, *J* = 7.2 Hz, 2H), 3.48 (ABq, 2H, Δ*ν*_{AB}=31.8 Hz, *J*_{AB}=10.0 Hz, -C*H*₂-O-), 3.05 (t, *J =* 7.2 Hz, 2H), 2.59 (t, *J* = 6.8 Hz, 2H), 2.15 (s, 3H, Ar-C*H*₃), 2.10 (s, 3H, Ar-C*H*₃), 2.09 (s, 3H, Ar-C*H*₃), 1.96 - 1.91 (m, 1H), 1.75 - 1.70 (m, 1H), 1.25 (s, 3H, -C*H*₃). ¹³C NMR (150 MHz, CDCl₃) *δ:* 145.2, 144.9, 144.4, 143.6, 128.8, 122.6, 121.5, 118.9, 117.5, 115.7, 115.4, 75.9, 74.9, 64.9, 52.2, 36.2, 28.8, 22.1, 21.9, 20.4, 12.1. MS m/z: 454.21 (M+H)⁺, 906.59 (2M)⁺, 928.89 (2M+Na)⁺. HRMS m/z: calcd for C₂₅H₃₂O₅N₃ [M+H]⁺ 454.23360, found for C₂₅H₃₂O₅N₃ 454.23389, calcd for C₂₃H₃₁O₅N₃Na [M+Na]⁺ 476.21560, found for C₂₅H₃₁O₅N₃Na 476.21552, calcd for C₂₃H₃₀O₅N₃ [M-H]⁻ 452.21910, found for C₂₅H₃₀O₅N₃ 452.21791.

### Example 3

**5-(3,4-Dihydro-6-hydroxy-2,5,7,8-tetramethyl-2*H*-benzopyran-2-yl)-3-(3,4-dihydroxyphenethyl)-isoxazole (18, MK153)** According to the general method for the preparation of the final hydroxy-analoques using the protected analoque **16** (0.05 g, 0.11 mmol), the desired product was obtained as white solid, (0.02 g, 47%). ¹H NMR (600 MHz, CDCl₃) *δ:* 6.64 (d, *J =* 8.0 Hz, 1H), 6.57 (d, *J =* 1.5 Hz, 1H), 6.44 (dd, *J =* 8.0, 1.4 Hz, 1H), 5.65 (s, 1H, *H*-isoxazole), 2.84 - 2.75 (m, 4H), 2.63 - 2.59 (m, 1H), 2.47 - 2.43 (m, 1H), 2.30 - 2.25 (m, 1H), 2.19 (s, 3H, Ar-C*H*₃), 2.18 (s, 3H, Ar-C*H*₃), 2.04 (s, 3H, Ar-C*H*₃), 2.02 - 1.99 (m, 1H), 1.67 (s, 3H, -C*H*₃). ¹³C NMR (75 MHz, CDCl₃) *δ:* 175.3, 163.5, 145.4, 145.2, 144.0, 142.3, 133.4, 122.6, 121.9, 120.8, 119.1, 117.4, 115.5, 115.5, 101.3, 74.4, 33.9, 31.6, 28.1, 27.7, 21.0, 12.5, 12.1, 11.6. MS m/z: 410.14 (M+H)⁺, 432.17 (M+Na)⁺, 840.95 (2M+Na)⁺. HRMS m/z: calcd for C₂₄H₂₈O₅N [M+H]⁺ 410.19620, found for C₂₄H₂₈O₅N 410.19633, calcd for C₂₄H₂₇O₅NNa [M+Na]⁺ 432.17811, found for C₂₄H₂₇O₅NNa 432.17834, calcd for C₂₄H₂₆O₅N [M-H]⁻ 408.18161, found for C₂₅H₃₀O₅N₃ 408.18076.

### Example 4

**5-{[(3,4-Dihydro-6-hydroxy-2,5,7,8-tetramethyl-2H-1-benzopyran-2-yl)methoxy]-methyl}-3-(3,4-dihydroxyphenethyl)-isoxazole (19, MK154)** According to the general method for the preparation of the final hydroxy-analoques using the protected analogue 17 (0.05 g, 0.10 mmol), the desired product was obtained as an oil, (0.02 g, 45%). ¹H NMR (600 MHz, CDCl₃) *δ:* 6.73 (d, *J =* 8.0 Hz, 1H), 6.65 (d, *J =* 1.9 Hz, 1H), δ 6.59 (dd, *J =* 8.1, 1.9 Hz, 1H), 5.97 (s, 1H, H-isoxazole), 4.65 (ABq, 2H, Δ*v*_{AB}=22.1 Hz, *J*_{AB}=14.0 Hz, -O-C*H*₂-), 3.52 (ABq, 2H, Δ*v*_{AB}=30.9 Hz, *J*_{AB}=9.9 Hz, -C*H*₂-O-), 2.93 - 2.90 (m, 2H), 2.85 - 2.83 (m, 2H), 2.63 - 2.60 (m, 2H), 2.16 (s, 3H, Ar-C*H*₃), 2.11 (bs, 6H, 2xAr-C*H*₃), 1.99 - 1.95 (m, 1H), 1.78 - 1.74 (m, 1H), 1.28 (s, 3H, -C*H*₃). ¹³C NMR (75 MHz, CDCl₃) *δ:* 169.2, 163.1, 148.5, 146.2, 145.1, 144.8, 143.6, 142.1, 133.3, 122.5, 120.6, 118.7, 117.2, 115.4, 115.3, 102.6, 76.0, 74.7, 64.5, 33.7, 28.5, 27.9, 21.9, 20.2, 12.2, 11.9, 11.3. MS m/z: 454.22 (M+H)⁺, 476.41 (M+Na)⁺, 906.81 (2M)⁺, 928.93 (2M+Na)⁺. HRMS m/z: calcd for C₂₆H₃₂O₆N [M+H]⁺ 454.22241, found for C₂₆H₃₂O₆N 454.22226, calcd for C₂₆H₃₁O₆NNa [M+Na]⁺ 476.20440, found for C₂₆H₃₁O₆NNa 476.20428, calcd for C₂₆H₃₀O₆N [M-H]⁻ 452.20790, found for C₂₆H₃₀O₆N 452.20671.

### Example 5

**2-(3,4-Dihydroxyphenethyl)-5-(3,4-dihydro-6-hydroxy-2,5,7,8-tetramethyl-2*H*-benzopyran-2-yl)-1,3,4-oxadiazole (22, MK155)** According to the general method for the preparation of the final hydroxy-analoques using the protected analoque **21** (0.07 g, 0.16 mmol), the desired product was obtained as gray solid, (0.04 g, 66%). ¹H NMR (300 MHz, CDCl₃) *6:* 6.49 (d, *J =* 8.1 Hz, 1H), 6.33 (d, *J =* 1.9 Hz, 1H), 6.19 (dd, *J = 8.1,* 1.7 Hz, 1H), 3.04 (t, *J =* 7.0 Hz, 2H), 2.87 - 2.75 (m, 2H), 2.70 - 2.61 (m, 3H), 2.17 (s, 3H, Ar-C*H*₃), 2.12 - 2.08 (m and s, 4H), 1.99 (s, 3H, Ar-C*H*₃), 1.73 (s, 3H, -C*H*₃).¹³C NMR (75 MHz, CDCl₃) *δ:* 168.7, 167.0, 145.7, 144.7, 144.0, 142.8, 130.9, 122.8, 122.1, 119.9, 119.4, 117.1, 115.1, 115.0, 72.3, 31.7, 30.7, 27.3, 26.8, 20.5, 12.3, 11.8, 11.3. MS m/z: 411.07 (M+H)⁺, 433.17 (M+Na)⁺, 820.77 (2M)⁺, 842.90 (2M+Na)⁺. HRMS m/z: calcd for C₂₃H₂₇O₅N₂ [M+H]⁺ 411.19141, found for C₂₃H₂₇O₅N₂ 411.19138, calcd for C₂₃H₂₆O₅N₂Na [M+Na]⁺ 433.17341, found for C₂₃H₂₆O₅N₂Na 433.17333, calcd for C₂₃H₂₅O₅N₂ [M-H]⁻ 409.17690, found for C₂₃H₂₅O₅N₂ 409,17592.

### Example 6

**2-(3-(3,4-Dihydroxyphenethyl)-1,2,4-oxadiazol-5-yl)-2,5,7,8-tetramethyl-2*H*-1-benzopyran-6-ol (26, MK160)** According to the general method for the preparation of the final hydroxy-analoques using the protected analoque 25 (0.04 g, 0.09 mmol), the desired product was obtained as yellowish solid, (0.02 g, 42%). ¹H NMR (300 MHz, CDCl₃) *δ*: 6.64 (d, *J =* 8.0 Hz, 1H), 6.49 - 6.44 (m, 2H), 2.95 - 2.85 (m, 4H), 2.71 - 2.54 (m, 3H), 2.19 (s, 3H, Ar-C*H*₃), 2.16 - 2.09 (m and 1s, 4H, 2.14 s, 3H, Ar-C*H*₃), 2.04 (s, 3H, Ar-C*H*₃), 1.77 (s, 3H, -C*H*₃). ¹³C NMR (150 MHz, CDCl₃) *δ:* 174.0, 169.9, 146.1, 145.5, 144.5, 142.6, 133.5, 123.5, 122.0, 121.3, 119.5, 117.3, 115.9, 114.5, 74.4, 32.9, 32.0, 28.6, 27.7, 21.1, 12.8, 12.5, 11.9. MS m/z: 411.00 (M+H)⁺, 433.13 (M+Na)⁺, 842.64 (2M+Na)⁺. HRMS m/z: calcd for C₂₃H₂₇O₅N₂ [M+H]⁺ 411.19140, found for C₂₃H₂₇O₅N₂ 411.19127, calcd for C₂₃H₂₆O₅N₂Na [M+Na]⁺ 433.17340, found for C₂₃H₂₆O₅N₂Na 433.17314, calcd for C₂₃H₂₅O₅N₂ [M-H]⁻ 409.17690 , found for C₂₃H₂₅O₅N₂ 409.17590. **Potassium phthalimide (27)** (Menichetti S. et al. Eur. J. Org. Chem. 2010, 2218-2225) To a solution of KOH 84% (169.7 mmol, 9.5 g) in absolute ethanol (100 mL), phthalimide (119 mmol, 17.5 g) was added and the resulting mixture was refluxed for 1 h. The reaction mixture was then cooled to ambient temperature and filtered under vacuum. Potassium phthalimide was obtained as a white solid (21.2 g, 96%), after filtration, washings with absolute EtOH and drying and was used in the next step without further purification.

***N*,*N*'-Dithiobisphthalimide (28)** (Menichetti S. et al. Eur. J. Org. Chem. 2010, 2218-2225) To an ice-cold suspension of **27** (50 mmol, 9.26 g) in dry CH₂Cl₂ (50 mL), S₂Cl₂ (25 mmol, 2 mL) was added dropwise. The resulting mixture was stirred at ambient temperature for 24h. The reaction mixture was filtered and the filtrate was evaporated under reduced pressure. The residue was washed with water, diethyl ether and a solution of MeOH/CHCl₃ (1:2) to afford N,N'-dithiobisphthalimide as an off white solid (5.26 g, 59%), which was used in the next step without further purification. ¹H NMR (600 MHz, CDCl₃): δ 7.96 - 7.95 (m, 4H, Ar), 7.83 - 7.82 (m, 4H, Ar) ¹³C NMR (150 MHz, CDCl₃): δ 166.7, 135.1, 132.4, 124.5.

### 2-((3-((t-Butyldimethylsilyl)oxy)-6-hydroxy-2,4,5-trimethylphenyl)thio)isoindoline-1,3-dione (33) was synthesized as described in Menichetti S. et al. Eur. J. Org. Chem. 2010, 2218-2225

**Phthalimidosulfenyl chloride (29)** To a solution of **28** (7.0 mmol, 2.5 g) in dry CH₂Cl₂ (70 mL) were added dry pyridine (0.3 mL) and SO₂Cl₂ (42 mmol, 3.4 mL) dropwise. The reaction mixture was stirred at ambient temperature for 48 h and evaporated to dryness. Phthalimidosulfenyl chloride **(29)** was obtained as a yellow solid which was used in the next step without further purification.

**4-Hydroxy-2,3,5-trimethylphenyl pivalate (30)** To an ice-cold solution of trimethylacetyl chloride (8.57 mmol, 1.05 mL) in dry CH₂Cl₂ (5 mL), was added a solution of trimethylhydroquinone (8.6 mmol, 1.30 g) and dry pyridine (20.8 mmol, 1.7 mL) in dry CH₂Cl₂ (4.7 mL). The reaction mixture was stirred at ambient temperature for 24 h. Upon completion of the reaction, the mixture was diluted with CH₂Cl₂, washed with a aq. AcOH solution (1M) and brine, dried over anhydrous Na₂SO₄ and evaporated to dryness. The desired compound **30** was obtained as an off white crystalline solid (1.4 g, 70%) after trituration of the residue with pentane. ¹H NMR (600 MHz, CDCl₃): δ 6.58 (s, 1H, Ar), 4.65 (s, 1H, OH), 2.17 (s, 3H, CH₃), 2.14 (s, 3H, CH₃), 2.02 (s, 3H, CH₃), 1.37 (2 s, 9H, C(CH₃)₃). ¹³C NMR (75 MHz, CDCl₃): δ 177.7, 149.7, 142.4, 127.0, 123.5, 121.3, 120.6, 39.2, 27.4, 15.9, 12.7, 12.3 HRMS (ESI): Calcd C₁₄H₂₁O₃ [M+H]⁺ 237.14124, found C₁₄H₂₁O₃ 237.14844, calcd C₁₄H₂₀O₃Na [M+Na]⁺ 259.14124, found C₁₄H₂₀O₃Na 259.13000.

**4-((*t*-Butyldimethylsilyl)oxy)-2,3,5-trimethylphenyl pivalate (31)** To a solution of pivalate derivative **30** (5.2 mmol, 1.24 g) in dry DMF (10.5 mL), were added imidazole (10.5 mmol, 710 mg) and t-butyldimethylsilyl chloride (10.5 mmol, 1.57 g). The reaction mixture was stirred at ambient temperature for 48 h. Upon completion of the reaction, the mixture was evaporated to dryness, the residue was dissolved in CH₂Cl₂ and the solution was washed with sat. aq. NH₄Cl solution. The organic layer was washed with brine, dried over anhydrous Na₂SO₄ and evaporated to dryness. Compound **31** was obtained as an off white crystalline solid (1.8 g, 99%) after flash-column chromatography purification (PE/EtOAc 99:1). ¹H NMR (600 MHz, CDCl₃): δ 6.60 (s, 1H, Ar), 2.16 (s, 3H, CH₃), 2.12 (s, 3H, CH₃), 1.99 (s, 3H, CH₃), 1.37 (s, 9H, OCC(CH₃)₃), 1.03 (s, 9H, (SiC(CH₃)₃), 0.16 (s, 6H, (CH₃)₂Si). ¹³C NMR (75 MHz, CDCl₃): δ 177.4, 149.4, 143.3, 128.5, 127.1, 126.7, 121.0, 39.2, 27.4, 26.2, 18.8, 17.8, 14.6, 13.0, 3.0. HRMS (ESI): Calcd C₂₀H₃₅O₃Si [M+H]⁺ 351.22772, found C₂₀H₃₅O₃Si 351.23469, calcd C₂₀H₃₄O₃SiNa [M+Na]⁺ 373.22772, found C₂₀H₃₄O₃SiNa 373.21656

**4-((*t*-Butyldimethylsilyl)oxy)-2,3,5-trimethylphenol (32)** To an ice-cold suspension of LiAlH₄ (5.2 mmol, 197 mg) in dry THF (5.2 mL), was added a solution of derivative **31** (2.6 mmol, 910 mg) in dry THF (7.3 mL). The reaction mixture was stirred at 0 °C for 25 minutes. Upon completion of the reaction, the excess amount of LiAlH₄ was quenched by the addition of a solution THF/H₂O 95:5 dropwise at 0 °C.

The reaction mixture was then diluted with ethyl acetate and anhydrous Na₂SO₄ was added. The mixture was filtered through a celite pad and the filtrate was evaporated in vacuo. Compound **32** was obtained as a yellow oil (0.67 g, 96%) after flash-column chromatography purification (PE/Acetone 96:4). ¹H NMR (600 MHz, CDCl₃): δ 6.44 (s, 1H, Ar), 4.28 (s, 1H, OH), 2.14 (s, 3H, CH₃Ar), 2.12 - 2.11 (2s, 6H, CH₃-Ar), 1.03 (s, 9H, (SiC(CH₃)₃), 0.14 (s, 6H, (CH₃)₂Si). ¹³C NMR (75 MHz, CDCl₃): δ 147.5, 145.6, 128.5, 126.3, 120.9, 114.6, 26.2, 18.8, 17.7, 14.6, 12.2, -3.1 HRMS (ESI): Calcd C₁₅H₂₅O₂Si [M-H]⁻ 265.17021, found C₁₅H₂₅O₂Si 265.16277

### 2-((3-((t-Butyldimethylsilyl)oxy)-6-hydroxy-2,4,5-trimethylphenyl)thio)isoindoline-1,3-dione (33)

To an ice-cold solution of compound **32** (11.5 mmol, 3.10 g) in dry CHCl₃ (23.3 mL) was added a solution of compound **29** (14.0 mmol, 2.99 g) in dry CHCl₃ (28 mL) dropwise. The reaction mixture was stirred at ambient temperature for 2 h. Upon completion of the reaction, the mixture was diluted with CH₂Cl₂ and washed with sat. aq. NaHCO₃ solution, brine, dried over anhydrous Na₂SO₄ and evaporated to dryness. Compound **33** was obtained as a yellow solid (4.68 g, 91%) after flash-column chromatography purification (CH₂Cl₂/PE 30:5). ¹H NMR (600 MHz, CDCl₃): δ 8.39 (s, 1H, OH), 7.89 - 7.87 (m, 2H, Ar), 7.77 - 7.74 (m, 2H, Ar), 2.71 (s, 3H, CH₃), 2.17 (s, 3H, CH₃), 2.12 (s, 3H, CH₃), 1.02 (s, 9H, SiC(CH₃)₃), 0.11 (s, 6H, (CH₃)₂Si). ¹³C NMR (150 MHz, CDCl₃): δ 169.1, 152.5, 145.0, 135.4, 134.9, 132.2, 131.8, 124.2, 122.5, 116.1, 26.2, 18.8, 16.9, 15.2, 13.2, -3.1. HRMS (ESI): Calcd C₂₃H₃₀NO₄SSi [M+H]⁺ 444.15866, found C₂₃H₃₀NO₄SSi 444.16599

**2-(3,4-Dihydroxyphenyl)acetic acid (34)** To an ice-cold solution of 2-(3,4-dimethoxyphenyl)acetic acid (10.2 mmol, 2.0 g) in dry CH₂Cl₂ (40 mL), BF₃S(CH₃)₂ (101.9 mmol, 10.7 mL) was added dropwise. The reaction mixture was then stirred at ambient temperature, in the dark, for 24 h. Upon completion of the reaction, the solvent was evaporated under nitrogen flow. The desired product **34** was obtained as a yellow oil and used to the next step without further purification.

**Methyl 2-(3,4-dihydroxyphenyl)acetate (35)** To a solution of **34** (10.2 mmol) in methanol (140 mL) conc. H₂SO₄ was added (10 drops). The reaction mixture was then refluxed, in the dark, for 2 h. Upon completion of the reaction, the mixture was cooled to ambient temperature and evaporated in vacuo almost to dryness. The residue was diluted with ethyl acetate and washed with sat. aq. NaHCO₃ solution. The organic layer was washed with brine, dried over anhydrous Na₂SO₄ and evaporated to dryness. The desired product **35** was obtained as a dark yellow oil which was used to the next step without further purification. ¹H NMR (300 MHz, CD₃OD): δ 6.70 - 6.68 (m, 2H, Ar), 6.56 (dd, *J =* 8.1, 1.9 Hz, 1H, Ar), 3.66 (s, 3H, OCH₃), 3.46 (s, 2H, CH₂)

**Methyl 2-(2,2-dimethylbenzo[d][1,3]dioxol-5-yl)acetate (36)** To a solution of **35** (10.2 mmol) in dry CH₂Cl₂ (102 mL), were added 2,2-dimethoxypropane (101.9 mmol, 12.5 mL) and camphorsulfonic acid (1.9 mmol, 438 mg). The reaction mixture was refluxed in the dark for 24 h. Upon completion of the reaction, the mixture was cooled to ambient temperature and sat. aq. NaHCO₃ solution was added to pH 7. The mixture was then diluted with ethyl acetate and the organic layer was washed with brine, dried over anhydrous Na₂SO₄ and evaporated to dryness. Compound **36** was obtained as a colorless oil (yield over 3 steps starting from 2-(3,4-dimethoxyphenyl)acetic acid: 2.1 g, 92%) after flash-column chromatography purification (PE/EtOAc 95:5). ¹HNMR (600 MHz, CDCl₃): δ 6.68 - 6.66 (m, 3H, Ar), 3.69 (s, 3H, OCH₃), 3.52 (s, 2H, CH₂), 1.66 (s, 6H, C(CH₃)₂). ¹³C NMR (75 MHz, CDCl₃): δ 172.3, 147.7, 146.6, 126.9, 121.8, 118.0, 109.6, 108.2, 52.1, 40.9, 25.95

**2-(2,2-Dimethylbenzo[d][1,3]dioxol-5-yl)ethan-1-ol (37)** To an ice-cold suspension of LiAlH₄ (14 mmol, 532 mg) in dry THF (20 mL), was added a solution of compound **36** (9.3 mmol, 2.08 g) in dry THF (32 mL) dropwise. The reaction mixture was stirred at ambient temperature for 1 h. Upon completion of the reaction, the excess amount of LiAlH₄ was quenched by the addition of a solution THF/H₂O 95:5 dropwise at 0 °C. The reaction mixture was then diluted with ethyl acetate and anhydrous Na₂SO₄ was added. The mixture was filtered through a celite pad and the filtrate was evaporated in vacuo. Compound **37** was obtained as a yellow oil and used in the next step without further purification. ¹H NMR (300 MHz, CDCl₃): δ 6.68 - 6.63 (m, 3H, Ar), 3.80 (t,*J* = 6.4 Hz, 2H, CH₂OH), 2.77 (t,*J* = 6.4 Hz, 2H, CH₂Ar), 1.66 (s, 6H, C(CH₃)₂). ¹³C NMR (75 MHz, CDCl₃): δ 147.7, 146.1, 131.5, 121.3, 117.8, 109.2, 108.2, 63.9, 38.97, 25.9

**2-(2,2-Dimethylbenzo[d][1,3]dioxol-5-yl)ethyl4-methylbenzenesulfonate (38)** To an ice-cold solution of compound **37** (9.3 mmol) in dry CH₂Cl₂ (38.1 mL), Et₃N (72.5 mmol, 10.2 mL) was added dropwise. Then a solution of *p*-toluenesulfonyl chloride (24.2 mmol, 4.61 g) in dry CH₂Cl₂ (20 mL) was added dropwise at 0 °C and the reaction mixture was stirred at ambient temperature for 19 h. Upon completion of the reaction, the mixture was diluted with CH₂Cl₂, washed with brine, dried over anhydrous Na₂SO₄ and evaporated to dryness. Compound **38** was obtained as a colorless crystalline solid (yield over 2 steps starting from **36:** 3.2 g, 99%) after flash-column chromatography purification (PE/EtOAc 9:1). ¹H NMR (600 MHz, CDCl₃): δ 7.72 (d, *J =* 8.2 Hz, 2H, Ar), 7.30 (d, *J =* 8.0 Hz, 2H, Ar), 6.59 (d, *J =* 7.8 Hz, 1H, Ar), 6.51 - 6.49 (m, 2H, Ar), 4.14 (t,*J* = 7.2 Hz, 2H, CH₂O), 2.84 (t, *J =* 7.2 Hz, 2H, CH₂Ar), 2.44 (s, 3H, CH₃), 1.65 (s, 6H, C(CH₃)₂). ¹³C NMR (75 MHz, CDCl₃): δ 147.6, 146.4, 144.7, 133.1, 129.8, 129.2, 127.9, 121.4, 117.9, 109.1, 108.2, 70.9, 35.2, 25.9, 21.7. HRMS (ESI): Calcd C₁₈H₂₁O₅S [M+H]⁺ 349.10314, found γ 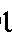 α C₁₈H₂₁O₅S 349.11054, calcd C₁₈H₂₀O₅SNa [M+Na]⁺ 371.10314, found C₁₈H₂₀O₅SNa 371.09211

**5-(2-Azidoethyl)-2,2-dimethylbenzo[d] [1,3]dioxole (39)** To a solution of compound 38 (2.5 mmol, 890 mg) in dry DMF (8.5 mL), NaN₃ (25.4 mmol, 1.66 g) was added. The reaction mixture was stirred at ambient temperature for 24 h. Upon completion of the reaction, the mixture was diluted with ethyl acetate, washed with brine, dried over anhydrous Na₂SO₄ and evaporated to dryness. Product **39** was obtained as a colorless oil and was used in the next step without further purification. ¹H NMR (600 MHz, CDCl₃): δ 6.67 - 6.60 (m, 3H, Ar), 3.45 (t, *J =* 7.2 Hz, 2H, CH₂N₃) 2.79 (t, *J =* 7.2 Hz, 2H, CH₂Ar), 1.66 (s, 6H, C(CH₃)₂). ¹³C NMR (150 MHz, CDCl₃): δ 147.7, 146.4, 131.1, 121.2, 117.9, 108.97, 108.3, 52.8, 35.2, 25.9

**1-(1-(2-(2,2-Dimethylbenzo[d][1,3]dioxol-5-yl)ethyl)-1*H*-1,2,3-triazol-4-yl)ethan-1-ol (40)** To a solution of compound **39** (2.2 mmol, 489 mg) in CH₂Cl₂ (6.7 mL), 3-butyn-2-ol (4.5 mmol, 0.35 mL) was added. Then, water (2.2 mL), copper (II) sulfate pentahydrate (0.67 mmol, 167 mg) and (+)-sodium L-ascorbate (1.3 mmol, 265 mg) were added to the solution. The reaction mixture was stirred vigorously at ambient temperature for 24 h. Upon completion of the reaction, the mixture was diluted with water and the organic layer washed with brine, dried over anhydrous Na₂SO₄ and evaporated to dryness. Compound **40** was obtained as an off green crystalline solid (yield over 2 steps starting from 38, 0.528 g, 82%) after flash-column chromatography purification (CH₂Cl₂/MeOH 95:5). ¹H NMR (300 MHz, CD₃OD): δ 7.69 (bs, 1H, triazole), 6.61 - 6.50 (m, 3H, Ar), 4.94 (bs, 1H, CH(OH)CH₃), 4.56 (t, *J = 7.1* Hz, 2H, CH₂N), 3.09 (t, *J =* 7.1 Hz, 2H, CH₂Ar), 1.61 (s, 6H, C(CH₃)₂), 1.49 (d, *J =* 5.8 Hz, 3H, CH₃). ¹³C NMR (75 MHz, CD₃OD): δ 149.0, 147.8, 131.8, 122.9, 122.3, 118.9, 109.8, 109.0, 63.7, 52.9, 37.3, 25.9, 23.8. HRMS (ESI): Calcd C₁₅H₂₀N₃O₃ [M+H]⁺ 290.14264, found C₁₅H₂₀N₃O₃ 290.14983, calcd C₁₅H₁₉N₃O₃Na [M+Na]⁺ 312.14264, found C₁₅H₁₉N₃O₃Na 312.13141

**1-(1-(2-(2,2-Dimethylbenzo[d][1,3]dioxol-5-yl)ethyl)-1*H*-1,2,3-triazol-4-yl)ethan-1-one (41)** To a solution of compound **40** (1.8 mmol, 514 mg) in dry CH₂Cl₂ (35.5 mL), activated MnO₂ (17.8 mmol, 1.55 g) was added. The reaction mixture was stirred at ambient temperature for 2.5 h. Upon completion of the reaction, the mixture was filtered through a celite pad and the filtrate was evaporated in vacuo. Compound **41** was obtained as a white solid (493 mg, 97%). ¹H NMR (300 MHz, CDCl₃): δ 7.81 (s, 1H, triazole), 6.59 (d, *J = 7.8* Hz, 1H, Ar), 6.47 - 6.43 (m, 2H, Ar), 4.57 (t, *J =* 7.1 Hz, 2H, CH₂N), 3.10 (t, *J =* 7.1 Hz, 2H, CH₂Ar), 2.65 (s, 3H, CH₃C=O), 1.63 (s, 6H, C(CH₃)₂). ¹³C NMR (75 MHz, CDCl₃): δ 192.9, 147.9, 147.85, 146.7, 129.3, 125.7, 121.1, 118.2, 108.6, 108.4, 52.2, 36.3, 27.1, 25.8. HRMS (ESI): Calcd C₁₅H₁₈N₃O₃ [M+H]⁺ 288.12699, found C₁₅H₁₈N₃O₃ 288.13424, calcd C₁₅H₁₇N₃O₃Na [M+Na]⁺ 310.12699, found C₁₅H₁₇N₃O₃Na 310.11594.

**1-(2-(2,2-Dimethylbenzo[d][1,3]dioxol-5-yl)ethyl)-4-(prop-1-en-2-yl)-1*H*-1,2,3-triazole (42)** To an ice-cold suspension of methyltriphenylphosphonium bromide (4.96 mmol, 1.77 g) in dry THF (3.3 mL), potassium tert-butoxide (4.96 mmol, 556 mg) was added at once. The reaction mixture was stirred at 0 °C for 15 minutes. Then, a solution of compound **41** (1.7 mmol, 474 mg) in dry THF (6.4 mL) was added dropwise and the reaction mixture was stirred at ambient temperature for 1 h. Upon completion of the reaction, the mixture was diluted with water and extracted with ethyl acetate. The organic layer was washed with brine, dried over anhydrous Na₂SO₄ and evaporated to dryness. Compound **42** was obtained as a yellow oil (461 mg, 98%) after flash-column chromatography purification (PE/Acetone 85:15). ¹H NMR (300 MHz, CDCl₃): δ 7.27 (s, 1H, triazole), 6.61 (d, *J = 7.6* Hz, 1H, Ar), 6.50 - 6.47 (m, 2H, Ar), 5.64 (s, 1H, CH₂=C), 5.05 - 5.04 (m, 1H, CH₂=C) 4.49 (t, *J =* 7.3 Hz, 2H, CH₂N), 3.07 (t, *J = 7.3* Hz, 2H, CH₂Ar), 2.08 (s, 3H, CH₃), 1.64 (s, 6H, C(CH₃)₂). ¹³C NMR (75 MHz, CDCl₃): δ 148.5, 147.8, 146.5, 133.6, 130.1, 121.2, 120.0, 118.1, 112.4, 108.9, 108.4, 51.9, 36.6, 25.9, 20.7 . HRMS (ESI): Calcd C₁₆H₂₀N₃O₂ [M+H]⁺ 286.14773, found C₁₆H₂₀N₃O₂ 286.15507, calcd C₁₆H₁₉N₃O₂Na [M+Na]⁺ 308.14773, found C₁₆H₁₉N₃O₂Na 308.13695

**4-(6-((*t*-Butyldimethylsilyl)oxy)-2,5,7,8-tetramethyl-2,3-dihydrobenzo[b][1,4]oxathiin-2-yl)-1-(2-(2,2-dimethylbenzo[d][1,3]dioxol-5-yl)ethyl)-1H-1,2,3-triazole (43 )**To an ice-cold solution of compound **33** (0.139 mmol, 61.7 mg) in dry CHCl₃ (2.8 mL), were added dry Et₃N (20 µL) and **42** (0.139 mmol, 39.7 mg). The reaction mixture was then refluxed for 48 h. Upon completion of the reaction, the solvent was evaporated under pressure to dryness. The residue was purified by flash-column chromatography purification (PE/EtOAc 83:17) to afford compound **43** as a dark yellow oil (53.5 mg, 66%). ¹H NMR (300 MHz, CDCl₃): δ 7.09 (s, 1H, triazole), 6.59 (d, *J = 7.8* Hz, 1H, Ar), 6.44 - 6.41 (m, 2H, Ar), 4.56 - 4.37 (m, 2H, CH₂N), 3.35, 3.21 (AB_{q}, *J_{AB} =* 13.0 Hz, 2H, CH₂S), 3.05 (t, *J =* 7.0, 2H, CH₂Ar), 2.11 (s, 3H, CH₃Ar), 2.10 (s, 3H, CH₃Ar), 2.07 (s, 3H, CH₃Ar), 1.77 (s, 3H, CH₃), 1.65 (s, 6H, (CH₃)₂C), 1.03 (s, 9H, SiC(CH₃)₃), 0.11 (d, *J =* 1.5, 6H, (CH₃)₂Si). ¹³C NMR (75 MHz, CDCl₃): δ 151.9, 147.8, 145.3, 142.3, 134.4, 132.7, 130.1, 124.8, 124.3, 123.7, 122.2, 121.2, 118.1, 114.6, 108.3, 72.2, 52.0, 36.6, 35.0, 27.2, 26.1, 25.9, 18.7, 14.4, 14.3, 12.4, 3.3. HRMS (ESI): Calcd C₃₁H₄₄N₃O₄SSi [M+H]⁺ 582.27435, found C₃₁H₄₄N₃O₄SSi 582.28169, calcd C₃₁H₄₃N₃O₄SSiNa [M+Na]⁺ 604.27435, found C₃₁H₄₃N₃O₄SSiNa 604.26364

**2-(1-(2-(2,2-Dimethylbenzo[d][1,3]dioxol-5-yl)ethyl)-1H-1,2,3-triazol-4-yl)-2,5,7,8-tetramethyl-2,3-dihydrobenzo [b] [1,4]oxathiin-6-ol (44)** To a solution of **43** (0.21 mmol, 122.2 mg) in dry THF (4.2 mL), a solution of TBAF in dry THF (4.2 mL, 0.05M) was added. The reaction mixture was stirred at ambient temperature for 40 minutes. Upon completion of the reaction, the mixture was diluted with CH₂Cl₂ and washed with sat. aq. NH₄Cl solution. The organic layer was washed with brine, dried over anhydrous Na₂SO₄ and evaporated to dryness. The residue was purified by flash-column chromatography (PE/EtOAc 75:25) to afford compound **44** as a white solid (71 mg, 72%). ¹H NMR (300 MHz, CDCl₃): δ 7.08 (s, 1H, triazole), 6.57 (d, *J =* 7.7 Hz, 1H, Ar), 6.45 - 6.39 (m, 2H, Ar), 4.55 - 4.38 (m, 2H, CH₂N), 3.37, 3.19 (AB_{q}, *J_{AB} =* 13.0 Hz, 2H, CH₂S), 3.05 (t, *J* = 6.9 Hz, 2H, CH₂Ar), 2.17 (s, 3H, CH₃Ar), 2.13 (s, 6H, 2CH₃Ar), 1.77 (s, 3H, CH₃), 1.65 (s, 6H, C(CH₃)₂). ¹³C NMR (75 MHz, CDCl₃): δ 151.8, 147.8, 146.5, 145.7, 141.9, 130.1, 124.2, 121.3, 121.2, 119.9, 118.1, 117.0, 114.6, 108.8, 108.3, 72.3, 52.0, 36.6, 35.0, 27.3, 25.9, 12.3. HRMS (ESI): Calcd C₂₅H₃₀N₃O₄S [M+H]⁺ 468.18788, found C₂₅H₃₀N₃O₄S 468.19481, Calcd C₂₅H₂₉N₃O₄SNa [M+Na]⁺ 490.18788, found C₂₅H₂₉N₃O₄SNa 490.17648, Calcd C₂₅H₂₈N₃O₄S [M-H]⁻ 466.18788, found C₂₅H₂₈N₃O₄S 466.17964

### Example 7

**4-(2-(4-(6-Hydroxy-2,5,7,8-tetramethyl-2,3-dihydrobenzo[b][1,4]oxathiin-2-yl)-1H-1,2,3-triazol-1-yl)ethyl)benzene-1,2-diol (45, TC369)** To an ice-cold solution of **44** (0.094 mmol, 44 mg) in degassed dry CHCl₃ (2 mL), a solution of trifluoroacetic acid/water 75:25 (0.65 mL) was added. The reaction mixture was stirred at ambient temperature for 24 h. Upon completion of the reaction, sat. aq. NaHCO₃ solution was added to pH 7. Then, the mixture was diluted with CHCl₃ and the organic layer was washed with brine, dried over anhydrous Na₂SO₄ and evaporated to dryness. The residue was purified by flash-column chromatography (CH₂Cl₂/MeOH 96:4) to afford compound **45** as a yellow oil (15.5 mg, 31%). ¹H NMR (600 MHz, CDCl₃): δ 7.08 (s, 1H, triazole), 6.71 (d, *J =* 1.7 Hz, 1H, Ar), 6.69 (d, *J =* 8.0 Hz, 1H, Ar), 6.39 (dd, *J =* 8.0, 1.7 Hz, 1H, Ar), 4.58 - 4.40 (m, 2H, CH₂N), 3.37, 3.18 (AB_{q}, *J_{AB} =* 13.0 Hz, 2H, CH₂S), 3.08 - 2.98 (m, 2H, CH₂Ar), 2.16 (s, 3H, CH₃Ar), 2.13 (s, 3H, CH₃Ar), 2.11 (s, 3H, CH₃Ar), 1.77 (s, 3H, CH₃). ¹³C NMR (150 MHz, CDCl₃): δ 151.6, 145.7, 144.4, 143.2, 142.0, 129.3, 124.4, 121.8, 120.7, 120.3, 117.3, 115.7, 115.4, 114.5, 72.2, 52.2, 36.3, 34.9, 27.9, 12.5, 12.4, 12.3. HRMS (ESI): Calcd C₂₂H₂₆N₃O₄S [M+H]⁺ 428.15658, found C₂₂H₂₆N₃O₄S 428.16338, Calcd C₂₂H₂₅N₃O₄SNa [M+Na]⁺ 450.15658, found C₂₂H₂₅N₃O₄SNa 450.14526, Calcd C₂₂H₂₄N₃O₄S [M-H]⁻ 426.15658, found C₂₂H₂₄Nς₃O₄S 426.14826

**3-(2,2-Dimethylbenzo[d] [1,3]dioxol-5-yl)propanenitrile (46)** To a solution of compound **38** (5.9 mmol, 2.05 g) in dry DMSO (5.9 mL), NaCN (17.6 mmol, 864.4 mg) was added. The reaction mixture was stirred at 50 °C for 1 h. Upon completion of the reaction, water was added at 0 °C and Et₂O. The organic layer was then washed with brine, dried over anhydrous Na₂SO₄ and evaporated to dryness. The residue was purified by flash-column chromatography (PE/Acetone 9:1) to afford compound **46** as a colorless oil (1.128g, 94%). ¹H NMR (600 MHz, (CD₃)₂CO): δ 6.75 - 6.68 (m, 3H, Ar), 2.85 (t, *J* = 7.3 Hz, 2H, CH₂CN), 2.70 (t, *J* = 7.3 Hz, 2H, CH₂Ar), 1.63 (s, 6H, C(CH₃)₂). ¹³C NMR (150 MHz, (CD₃)₂CO): δ 148.5, 147.2, 133.0, 121.8, 120.2, 118.6, 109.3, 108.8, 31.8, 25.9, 19.6. HRMS (ESI): Calcd C₁₂H₁₄NO₂ [M+H]⁺ 204.09463, found C₁₂H₁₄NO₂ 204.10174, Calcd C₁₂H₁₃NO₂Na [M+Na]⁺ 226.09463, found C₁₂H₁₃NO₂Na 226.08333

**3-(2,2-Dimethylbenzo[d][1,3]dioxol-5-yl)propanal (47)** To a solution of **46** (0.69 mmol, 140 mg) in dry CH₂Cl₂ (2.85 mL), a solution of DIBAL-H (1M in hexane) (0.9 mL) was added dropwise at -78 °C and the reaction mixture was stirred at -78°C for 1.5 h. Then, an aq. solution of HCl 2M (2.5 mL) was added and the resulting mixture was stirred at ambient temperature for 30 minutes. The mixture was diluted with CH₂Cl₂ and the organic layer was washed with aq. HCl (2M), brine, dried over anhydrous Na₂SO₄ and evaporated to dryness. The oily residue, compound **47** was used in the next step without further purification. ¹H NMR (600 MHz, CDCl₃): δ 9.81 (t, *J =* 1.4 Hz, 1H, CHO), 6.64 - 6.58 (m, 3H, Ar), 2.86 (t, *J =* 7.5 Hz, 2H, CH₂CHO), 2.73 (t, *J =* 7.5 Hz, 2H, CH₂Ar), 1.66 (s, 6H, C(CH₃)₂). HRMS (ESI): Calcd C₁₂H₁₅O₃ [M+H]⁺ 207.09429, found C₁₂H₁₅O₃ 207.10155, Calcd C₁₂H₁₄O₃Na [M+Na]⁺429.09429, found C₁₂H₁₄O₃Na 429.08345

**(Z)-3-(2,2-Dimethylbenzo[d] [1,3]dioxol-5-yl)propanal oxime (48)** To a solution of **47** (0.64 mmol, 132 mg) in *t*-butanol/H₂O 1:1 (2.6 mL), were added NH₂OH·HCl (0.96 mmol, 62 mg) and aq. NaOH (1N) (0.96 mL). The reaction mixture was stirred at ambient temperature for 1.5 h. Upon completion of the reaction, the mixture was extracted with ethyl acetate. The organic layer was washed with brine, dried over anhydrous Na₂SO₄ and evaporated to dryness. The residue was purified by flash-column chromatography (PE/EtOAc 9:1) to afford compound **48** as colorless crystalline solid (127.7 mg, 90%), mixture of geometrical isomers. ¹H NMR (600 MHz, CDCl₃): δ 7.45 (t, 0.5H, CH=N), 6.74 (t, 0.5H, CH=N), 6.65 - 6.58 (m, 3H, Ar), 2.74 - 2.71 (m, 2H, CH₂), 2.67 - 2.66 (m, 1H, CH₂), 2.49 - 2.45 (m, 1H, CH₂), 1.66 (s, 6H, C(CH₃)₂). ¹³C NMR (MHz, CDCl₃): δ 150.7, 150.0, 148.4, 146.6, 135.4, 121.3, 109.2, 108.6, 32.5, 27.4, 25.9. HRMS (ESI): Calcd C₁₂H₁₆NO₃ [M+H]⁺ 222.10519, found C₁₂H₁₆NO₃ 222.11238, Calcd C₁₂H₁₅NO₃Na [M+Na]⁺ 244.10519, found C₁₂H₁₅NO₃Na 244.09422, Calcd C₁₂H₁₄NO₃ [M-H]⁻ 220.10519, found C₁₂H₁₄NO₃ 220.09775

**1-(3-(2-(2,2-Dimethylbenzo[d][1,3]dioxol-5-yl)ethyl)isoxazol-5-yl)ethan-1-one (49)** To a solution of **48** (0.32 mmol, 70 mg) in dry butanone (2.65 mL), were added 3-butyn-2-one (0.47 mmol, 32.3 mg) and isopentyl nitrite (0.35 mmol, 40.7 mg). The reaction mixture was stirred in ambient temperature for 5 minutes and then was refluxed for 1h. Upon completion of the reaction, the mixture was cooled to ambient temperature and the solvent was evaporated in vacuo to dryness. Purification of the residue by flash-column chromatography (PE/EtOAc 9:1) afforded **49** as a yellow oil (25 mg, 27%). ¹H NMR (600 MHz, CDCl₃): δ 6.66 - 6.56 (m, 3H Ar, 1H oxazole), 3.01 (t, *J =* 7.8 Hz, 2H, CH₂- oxazole), 2.90 (t, *J =* 7.8 Hz, 2H, CH₂Ar), 2.59 (s, 3H, CH₃), 1.66 (s, 6H, C(CH₃)₂). ¹³C NMR (75MHz, CDCl₃): δ 187.2, 166.6, 164.3, 147.8, 146.2, 133.2, 120.7, 118.0, 108.7, 108.3, 107.2, 34.2, 28.2, 27.4, 26.0. HRMS (ESI): Calcd C₁₆H₁₈NO₄ [M+H]⁺ 288.11576, found C₁₆H₁₈NO₄ 288.12292, Calcd C₁₆H₁₇NO₄Na [M+Na]⁺ 310.11576, found C₁₆H₁₇NO₄Na 310.10455

**3-(2-(2,2-Dimethylbenzo[d][1,3]dioxol-5-yl)ethyl)-5-(prop-1-en-2-yl)isoxazole (50)** To an ice-cold suspension of methyltriphenylphosphonium bromide (0.5 mmol, 180.2 mg) in dry THF (0.5 mL), potassium t-butoxide (0.5 mmol, 56.6 mg) was added at once. The reaction mixture was stirred at 0 °C for 15 minutes. Then, a solution of **49** (0.168 mmol, 48.3 mg) in dry THF (0.5 mL) was added dropwise and the reaction mixture was stirred at ambient temperature for 1 h. Upon completion of the reaction, the mixture was diluted with water and extracted with ethyl acetate. The organic layer was washed with brine, dried over anhydrous Na₂SO₄ and evaporated to dryness. Purification of the residue by flash-column chromatography (PE/EtOAc 95:5) afforded **50** as a yellow oil (32 mg, 67%). ¹HNMR (600 MHz, CDCl₃): δ 6.65 - 6.60 (m, 3H, Ar), 5.99 (s, 1H, oxazole), 5.74 (s, 1H CH₂=C), 5.26 (s, 1H, CH₂=C), 2.93 - 2.88 (m, 4H, 2CH₂), 2.05 (s, 3H, CH₃), 1.66 (s, 6H, C(CH₃)₂). ¹³C NMR (75MHz, CDCl₃): 170.2, 163.5, 147.5, 145.8, 133.8, 130.7, 120.5, 117.6, 116.6, 108.6, 108.0, 100.2, 34.2, 28.3, 25.8, 19.6. HRMS (ESI): Calcd C₁₇H₂₀NO₃ [M+H]⁺ 286.13649, found C₁₇H₂₀NO₃ 286.14381, Calcd C₁₇H₁₉NO₃Na [M+Na]⁺ 308.13649, found C₁₇H₁₉NO₃Na 308.12567

**5-(6-((*t*-Dutyldimethylsilyl)oxy)-2,5,7,8-tetramethyl-2,3-dihydrobenzo[b][1,4]oxathiin-2-yl)-3-(2-(2,2-dimethylbenzo[d][1,3]dioxol-5-yl)ethyl)isoxazole (51)** To an ice-cold solution of **33** (0.22 mmol, 98 mg) in dry CHCl₃ (4.4 mL), were added dry Et₃N (0.221 mmol, 31 µL) and compound **50** (0.11 mmol, 31.5 mg). The reaction mixture was then refluxed for 48 h and the solvent was evaporated in vacuo. The residue was purified by flash-column chromatography (PE/EtOAc 95:5) to afford compound **51** along with starting material **50**, as a dark yellow oil.

**2-(3-(2-(2,2-Dimethylbenzo[d][1,3]dioxol-5-yl)ethyl)isoxazol-5-yl)-2,5,7,8-tetramethyl-2,3-dihydro benzo[b][1,4]oxathiin-6-ol (52)** To a solution of **51** (0.11 mmol) in dry THF (1.6 mL), a solution of TBAF in dry THF (1.6 mL, 0.05M) was added. The reaction mixture was then stirred at ambient temperature for 40 minutes. Upon completion of the reaction, the mixture was diluted with CH₂Cl₂ and washed with sat. aq. NH₄Cl solution. The organic layer was washed with brine, dried over anhydrous Na₂SO₄ and evaporated in vacuo. The residue was purified by flash-column chromatography (PE/Acetone 9:1) to afford compound **52** as a yellow oil (yield over 2 steps starting from **50:** 21.6 mg, 42%). ¹H NMR (300 MHz, CDCl₃): δ 6.61 - 6.51 (m, 3H, Ar), 5.87 (s, 1H, oxazole), 4.47 (s, 1H, OH), 3.32, 3.13 (AB_{q}, *J_{AB} =* 13.1 Hz, 2H, CH₂S), 2.91 - 2.82 (m, 4H, 2CH₂), 2.17 (s, 6H, 2CH₃ Ar), 2.13 (s, 3H, CH₃ Ar), 1.76 (s, 3H, CH₃), 1.65 (s, 6H, C(CH₃)₂). ¹³C NMR (75 MHz, CDCl₃): δ 173.5, 163.3, 147.6, 146.1, 145.9, 141.7, 133.9, 124.5, 120.7, 120.3, 117.8, 117.2, 114.3, 108.7, 108.1, 101.3, 72.9, 34.2, 33.8, 28.5, 26.4, 26.0, 12.4. HRMS (ESI): Calcd C₂₆H₃₀NO₅S [M+H]⁺ 468.17664, found C₂₆H₃₀NO₅S 468.18372, Calcd C₂₆H₂₉NO₅S Na [M+Na]⁺ 490.17664, found C₂₆H₂₉NO₅S Na 490.16565

### Example 8

**4-(2-(5-(6-Hydroxy-2,5,7,8-tetramethyl-2,3-dihydrobenzo[b] [1,4]oxathiin-2-yl)isoxazol-3-yl)ethyl) benzene-1,2-diol (53, TC391)** To compound **52** (0.033 mmol, 15.6 mg), was added trifluoroacetic acid (0.5 mL) at 0 °C. The reaction mixture was stirred at ambient temperature for 30 minutes. Upon completion of the reaction, the mixture was diluted with toluene and the excess amount of trifluoroacetic acid was evaporated in vacuo. The residue was purified by flash-column chromatography (PE/Acetone 9:1) to afford compound **53** as a yellow oil (9 mg, 63%). ¹H NMR (600 MHz, CDCl₃): δ 6.68 (d, J = 5.8 Hz, 1H, Ar), 6.61 (s, 1H, Ar), 6.50 (d, *J* = 7.5 Hz, 1H, Ar), 5.79 (s, 1H, oxazole), 3.32, 3.14 (AB_{q}, *J_{AB} =* 13.1 Hz, 2H, CH₂S), 2.86 - 2.80 (2m, 4H, 2CH₂), 2.17 (2s, 6H, (CH₃)₂Ar), 2.12 (s, 3H, CH₃Ar), 1.76 (s, 3H, CH₃). ¹³C NMR (150MHz, CDCl₃): δ 173.5, 163.4, 145.9, 143.8, 142.2, 141.8, 133.3, 124.5, 120.8, 120.6, 117.4, 115.4, 114.2, 101.6, 72.8, 33.8, 33.7, 28.0, 26.7, 12.44, 12.38, 12.3. HRMS (ESI): Calcd C₂₃H₂₆NO₅S [M+H]⁺ 428.14534, found C₂₃H₂₆NO₅S 428.15255, Calcd C₂₃H₂₅NO₅SNa [M+Na]⁺ 450.14534, found C₂₃H₂₅NO₅SNa 450.13443, Calcd C₂₃H₂₄NO₅S [M-H]⁻ 426.14534, found C₂₃H₂₄NO₅S 426.13693

**(E,Z)-3-(2,2-Dimethylbenzo[d][1,3]dioxol-5-yl)-N'-hydroxypropanimidamide (54)** To a suspension of NH₂OH·HCl (2.2 mmol, 154 mg) in isopropanol (5.3 mL), NaHCO₃ (3.3 mmol, 273 mg) was added. The resulting mixture was stirred at ambient temperature for 20 minutes. Then, a solution of **46** (0.3 g, 1.5 mmol), in isopropanol (4.5 mL) was added and the reaction mixture was refluxed for 24 h. Upon completion of the reaction, the mixture was cooled to ambient temperature, filtered under vacuum and the filtrate was evaporated in vacuo. The residue was purified by flash-column chromatography (CH₂Cl₂/MeOH 96:4) to afford compound **54** as a colorless gummy solid (289 mg, 83%). ¹H NMR (600 MHz, CDCl₃): δ 6.64 - 6.61 (m, 3H, Ar), 4.50 (s, 2H, NH₂), 2.79 (t, J = 7.9, 2H, CH₂C), 2.40 (t, J = 7.9, 2H, CH₂Ar), 1.66 (s, 6H, C(CH₃)₂). ¹³C NMR (150 MHz, CDCl₃): δ 153.8, 147.6, 146.0, 133.9, 120.6, 117.8, 108.7, 108.2, 33.5, 32.9, 25.9. HRMS (ESI): Calcd C₁₂H₁₇N₂O₃ [M+H]⁺ 237.11609, found C₁₂H₁₇N₂O₃ 237.12336, Calcd C₁₂H₁₆N₂O₃Na [M+Na]⁺259.11609, found C₁₂H₁₆N₂O₃Na 259.10510

**3-(2-(2,2-Dimethylbenzo[d][1,3]dioxol-5-yl)ethyl)-5-(prop-1-en-2-yl)-1,2,4-oxadiazole (55)** In a 10 mL microwave tube were added a solution of methacrylic acid (0.25 mmol, 22 mg) in dry THF (0.9 mL), 2-chloro-4,6-dimethoxy-1,3,5-triazine (0.3 mmol, 54 mg) and N-methylmorpholine (0.8 mmol, 84 µL). The reaction mixture was stirred at ambient temperature for 1 h. After consumption of the acid (monitored with TLC), a solution of **54** (0.2 mmol, 50 mg), in dry toluene (0.4 mL) was added, and the mixture was heated under microwave irradiation (180 watt, 160 °C), for 6 minutes. The resulting mixture was cooled to ambient temperature, diluted with ethyl acetate, washed with brine, dried over anhydrous Na₂SO₄ and evaporated to dryness. Purification of the residue by flash-column chromatography (PE/EtOAc 97:3) afforded compound **55** as colorless oil (34 mg, 56%). ¹H NMR (600 MHz, CD₃OD): δ 6.62 - 6.60 (m, 3H, Ar), 6.21 (s, 1H, CH₂C), 5.72 (s, 1H, CH₂C), 2.96 (bs, 4H, CH₂CH₂), 2.19 (s, 3H, CH₃), 1.61 (s, 6H, C(CH₃)₂). ¹³C NMR (75 MHz, CD₃OD): δ 177.4, 171.5, 148.9, 147.3, 134.8, 130.9, 124.7, 121.8, 118.7, 109.5, 108.9, 33.8, 29.2, 25.9, 19.0. HRMS (ESI): Calcd C₁₆H₁₉N₂O₃ [M+H]⁺ 287.13174, found C₁₆H₁₉N₂O₃ 287.13894, Calcd C₁₆H₁₈N₂O₃Na [M+Na]⁺309.13174, found C₁₆H₁₈N₂O₃Na 309.12071.

**5-(6-((*t*-Butyldimethylsilyl)oxy)-2,5,7,8-tetramethyl-2,3-dihydrobenzo[b] [1,4] oxathiin-2-yl)-3-(2-(2,2-dimethylbenzo[d] [1,3]dioxol-5-yl)ethyl)-1,2,4-oxadiazole (56)** To an ice-cold solution **of33** (0.126 mmol, 55.8 mg) in dry CHCl₃ (2.5 mL), were added dry Et₃N (0.14 mmol, 20 µL) and **55** (0.105 mmol, 30 mg). The reaction mixture was then refluxed for 48 h. The solvent was evaporated in vacuo and product **56** was obtained with starting material **55,** as a dark yellow oil, after flash-column chromatography purification of the residue (Hexane/Acetone 98:2). The mixture was used in the next step without further purification.

**2-(3-(2-(2,2-Dimethylbenzo[d][1,3]dioxol-5-yl)ethyl)-1,2,4-oxadiazol-5-yl)-2,5,7,8-tetramethyl-2,3-dihydrobenzo[b][1,4]oxathiin-6-ol (57)** To a solution of **56** (0.105 mmol) in dry THF (1.1 mL), a solution of TBAF in dry THF (1.1 mL, 0.05M) was added. The reaction mixture was then stirred at ambient temperature for 40 minutes. Upon completion of the reaction, the mixture was diluted with CH₂Cl₂ and washed with sat. aq. NH₄Cl solution. The organic layer was washed with brine, dried over anhydrous Na₂SO₄ and evaporated in vacuo. The residue was purifie by flash-column chromatography (PE/Acetone 9:1) to afford compound **57** as a yellow oil (yield over 2 steps starting from **55:** 16.4 mg, 33%). ¹H NMR (600 MHz, CDCl₃): δ 6.59 (d, *J = 7.7* Hz, 1H, Ar), 6.55 - 6.53 (m, 2H, Ar), 3.50, 3.19 (AB_{q}, *J_{AB}* = 13.1 Hz, 2H, CH₂S), 2.99 - 2.92 (m, 4H, CH₂CH₂), 2.17 (s, 3H, CH₃Ar), 2.15 (s, 3H, CH₃Ar), 2.13 (s, 3H, CH₃Ar), 1.86 (s, 3H, CH₃), 1.65 (s, 6H, (CH₃)₂C). ¹³C NMR (150 MHz, CDCl₃): δ 179.8, 170.1, 147.6, 146.4, 146.0, 142.0, 133.4, 125.1, 120.7, 120.6, 117.8, 117.2, 114.2, 108.8, 108.2, 73.6, 34.1, 32.9, 28.5, 26.5, 26.0, 12.4. HRMS (ESI): Calcd C₂₅H₂₉N₂O₅S [M+H]⁺ 469.17189, found C₂₅H₂₉N₂O₅S 469.17906, Calcd C₂₅H₂₈N₂O₅S Na [M+Na]⁺ 491.17189, found C₂₅H₂₈N₂O₅SNa 491.16096

### Example 9

**4-(2-(5-(6-Hydroxy-2,5,7,8-tetramethyl-2,3-dihydrobenzo[b] [1,4]oxathiin-2-yl)-1,2,4-oxadiazol-3-yl)ethyl)benzene-1,2-diol (58, TC392)** To compound **57** (0.035 mmol, 16.4 mg), trifluoroacetic acid (1.5 mL) was added at 0 °C. The reaction mixture was stirred at ambient temperature for 30 minutes. Upon completion of the reaction, the mixture was diluted with toluene and the excess trifluoroacetic acid was evaporated in vacuo. The residue was purified by flash-column chromatography (Hexane/Acetone 7:3) to afford compound **58,** as a yellow oil (8.1 mg, 54%). ¹H NMR (600 MHz, CDCl₃): δ 6.69 (d, *J =* 8.0 Hz, 1H, Ar), 6.56 (s, 1H, Ar), 6.53 (d, *J = 8.0* Hz, 1H, Ar), 3.51, 3.21 (AB_{q}, *J_{AB} =* 13.1 Hz, 2H, CH₂S), 2.96 - 2.89 (2m, 4H, CH₂CH₂), 2.20 (s, 3H, CH₃Ar), 2.14 (s, 3H, CH₃Ar), 2.12 (s, 3H, CH₃Ar), 1.87 (s, 3H, CH₃). ¹³C NMR (150 MHz, CDCl₃): δ 179.8, 170.0, 146.3, 143.7, 142.3, 142.1, 133.1, 125.2, 120.9, 120.7, 117.2, 115.5, 115.4, 114.1, 73.6, 34.0, 32.4, 28.2, 26.7, 12.4. HRMS (ESI): Calcd C₂₂H₂₅N₂O₅S [M+H]⁺ 429.14059, found C₂₂H₂₅N₂O₅S 429.14772, Calcd C₂₂H₂₄N₂O₃SNa [M+Na]⁺ 451.14059, found C₂₂H₂₄N₂O₃SNa 451.12960

**Ethyl 3-(3,4-dimethoxyphenyl)propanoate (59)** To a solution of 3-(3,4-dimethoxyphenyl)propanoic acid (4.76 mmol, 1 g) in absolute ethanol (20 mL), conc. H₂SO₄ was added (10 drops). The reaction mixture was then refluxed for 4 h. Upon completion of the reaction, the mixture was cooled to ambient temperature and evaporated in vacuo. Et₂O was added to the residue and the mixture was washed with sat. aq. NaHCO₃ solution. The organic layer was washed with brine, dried over anhydrous Na₂SO₄ and evaporated in vacuo to afford compound **59** as a yellow oil (1.13 g, quantitative yield) which was used in the next step without further purification. ¹H NMR (600 MHz, CDCl₃): δ 6.79 (d, 8.3 Hz, 1H, Ar) 6.75 - 6.73 (m, 2H, Ar), 4.13 (q, *J =* 7.1 Hz, 2H, OCH₂), 3.86 (s, 3H, OCH₃), 3.85 (s, 3H, OCH3), 2.90 (t, *J =* 7.8 Hz, 2H, CH₂COO), 2.60 (t, *J =* 7.8 Hz, 2H, CH₂Ar), 1.24 (t*, J =* 7.2 Hz, 2H, CCH₃). ¹³C NMR (MHz, CDCl₃): δ 173.1, 149.0, 147.6, 133.4, 120.3, 111.8, 111.4, 60.5, 56.1, 56.0, 36.4, 30.8, 14.4. HRMS (ESI): Calcd C₁₃H₁₉O₄ [M+H]⁺ 239.12051, found C₁₃H₁₉O₄ 239.12781, Calcd C₁₃H₁₈O₄Na [M+Na]⁺261.10936, found C₁₃H₁₈O₄Na 261.10936

**3-(3,4-Dimethoxyphenyl)propanehydrazide (60)** In a 10 mL microwave tube were added a solution of compound **59** (1.15 mmol, 258 mg) in absolute ethanol (0.2 mL) and NH₂NH₂·H₂O (4.03 mmol, 0.45 mL). The reaction mixture was heated under microwave irradiation (180 watt, 120 °C) for 12 minutes. Upon completion, the mixture was cooled to ambient temperature, and the precipitate was collected by suction filtration, washed with absolute ethanol and dried under high vacuum to afford compound **60** as white solid (106 mg, 44%), which was used in the next step without further purification. ¹H NMR (600 MHz, CDCl₃): δ 6.79 (d, *J* = 7.8 Hz, 1H, Ar), 6.73 - 6.71 (m, 2H), 6.62 (s, 1H, NH), 3.86 - 3.85 (2s, 6H), 2.91 (t, *J* = 7.6 Hz, 2H), 2.43 (t, *J* = 7.6 Hz, 2H), 1.62 (s, 1H, NH₂). ¹³C NMR (MHz, CDCl₃): δ 173.1, 149.1, 147.8, 133.2, 120.2, 111.8, 111.5, 56.1, 56.0, 36.8, 31.3

**N'-(3-(3,4-Dimethoxyphenyl)propanoyl)methacrylohydrazide (61)** To an ice-cold solution of methacrylic acid (0.2 mmol, 19.3 mg), in dry THF (1 mL) were added compound **60** (0.27 mmol, 60 mg), HATU (0.34 mmol, 127.8 mg) and *N,N*-diisopropylethylamine (0.67 mmol, 0.12 mL) dropwise. The reaction mixture was stirred at ambient temperature for 2 h. Upon completion of the reaction, water was added at 0 °C and the mixture was extracted with ethyl acetate. The organic layer was washed with brine, dried over anhydrous Na₂SO₄ and evaporated in vacuo. The residue was purified by flash-column chromatography (PE/EtOAc 6:4) to afford compound **61** as a colorless oil (58.4 mg, 89%). ¹H NMR (600 MHz, CDCl₃): δ 8.87 (d, *J* = 5.5 Hz, 1H, NH), 8.72 (d, *J* = 5.6 Hz, 1H, NH), 6.78 (d, *J* = 7.8 Hz, 1H, Ar), 6.73 (d, *J* = 7.6 Hz, 2H, Ar), 5.85 (s, 1H, CH₂=C), 5.44 (s, 1H, CH₂=C), 3.85 (d, *J* = 5.8 Hz, 6H, 2OCH₃), 2.94 (t, *J* = 7.7 Hz, 2H, CH₂CO), 2.59 (t, *J* = 7.7 Hz, 2H, CH₂Ar), 1.98 (s, 3H, CH₃). ¹³C NMR (150 MHz, CDCl₃): δ 168.9, 164.5, 149.1, 147.7, 136.9, 132.9, 122.2, 120.2, 111.8, 111.5, 56.1, 56.0, 36.2, 31.1, 18.3. HRMS (ESI): Calcd C₁₅H₂₁N₂O₄ [M+H]⁺ 293.14231, found C₁₅H₂₁N₂O₄ 293.14970, Calcd C₁₅H₂₀N₂O₄Na [M+Na]⁺ 315.14231, found C₁₅H₂₀N₂O₄Na 315.13111, Calcd C₁₅H₁₉N₂O₄ [M-H]⁻ 291.14231, found C₁₅H₁₉N₂O₄ 291.13441

**2-(3,4-Dimethoxyphenethyl)-5-(prop-1-en-2-yl)-1,3,4-oxadiazole (62)** To an ice-cold solution of **61** (0.176 mmol, 51 mg) in dry CH₂Cl₂ (1.0 mL) were added 4-toluenesulfonyl chloride (0.53 mmol, 100.4 mg) and dry Et₃N (0.7 mmol, 0.1 mL) dropwise. The reaction mixture was stirred at ambient temperature for 24 h. Upon completion of the reaction, water was added at 0 °C and the mixture was extracted with CH₂Cl₂. The organic layer was washed with brine, dried over anhydrous Na₂SO₄ and evaporated in vacuo. Purification of the residue by flash-column chromatography (CH₂Cl₂/MeOH 97:3) afforded compound **62** as a colorless oil (43.2 mg, 90%). ¹H NMR (600 MHz, CDCl₃): *δ* 6.81 - 6.73 (m, 3H, Ar), 5.90 (s, 1H, CH₂=C), 5.49 (s, 1H, CH₂=C), 3.86 (s, 6H, 2OCH₃), 3.15 (t, *J* = 7.7 Hz, 2H, CH₂ - oxadiazole), 3.07 (t, J= 7.7 Hz, 2H, CH₂Ar), 2.20 (s, 3H, CH₃). ¹³C NMR (150 MHz, CDCl₃): δ 166.2, 165.5, 149.2, 148.0, 132.3, 129.1, 120.5, 120.4, 111.8, 111.6, 56.1, 56.0, 32.5, 27.8, 18.8. HRMS (ESI): Calcd C₁₅H₁₉N₂O₃ [M+H]⁺ 275.13174, found C₁₅H₁₉N₂O₃ 275.13907, Calcd C₁₅H₁₈N₂O₃Na [M+Na]⁺ 297.13174, found C₁₅H₁₈N₂O₃Na 297.12065

**2-(6-((*t*-Butyldimethylsilyl)oxy)-2,5,7,8-tetramethyl-2,3-dihydrobenzo[b][1,4]oxathiin-2-yl)-5-(3,4-dimethoxyphenethyl)-1,3,4-oxadiazole (63)** To an ice-cold solution of **33** (0.22 mmol, 98.1 mg), and **62** (0.66 mmol, 182 mg), in dry CHCl₃ (2.2 mL), dry Et₃N (0.22 mmol, 31 µL) was added. The reaction mixture was then refluxed for 24 h. The solvent was evaporated in vacuo and the product **63** was obtained along with starting material **62,** as a dark yellow oil, after flash-column chromatography purification of the residue (CH₂Cl₂/MeOH 98:2). The mixture was used in the next step without further purification. ¹H NMR (600 MHz, CDCl₃): δ 6.75 (d, *J* = 8.2 Hz, 1H, Ar), 6.70 (d, *J* = 1.8 Hz, 1H, Ar), 6.65 (dd, *J* = 8.1, 1.8 Hz, 1H, Ar), 3.84 (s, 6H, 2OCH₃), 3.51, 3.16 (AB_{q}, *J_{AB}* = 13.1 Hz, 2H, CH₂S), 3.13 (t, 2H, CH₂ oxadiazole), 3.03 - 3.00 (m, 2H, CH₂Ar), 2.12 (s, 3H, CH₃Ar), 2.101 (s, 3H, CH₃Ar), 2.096 (s, 3H, CH₃Ar), 1.84 (s, 3H, CH₃), 1.03 (s, 9H, C(CH₃)₃), 0.11 (s, 6H, Si(CH₃)₃). ¹³C NMR (150 MHz, CDCl₃): δ 167.7, 166.9, 149.2, 148.0, 146.0, 142.3, 132.1, 125.5, 125.0, 122.5, 120.4, 114.8, 111.7, 111.5, 72.2, 56.1, 56.0, 33.8, 32.3, 27.8, 26.2, 25.5, 18.7, 14.5, 14.4, 12.5, -3.17, -3.20. HRMS (ESI): Calcd C₃₀H₄₃N₂O₅SSi [M+H]⁺ 571.25837, found C₃₀H₄₃N₂O₅SSi 571.26578, Calcd C₃₀H₄₂N₂O₅SSiNa [M+Na]⁺ 593.25837, found C₃₀H₄₂N₂O₅SSiNa 593.24768

**2-(5-(3,4-Dimethoxyphenethyl)-1,3,4-oxadiazol-2-yl)-2,5,7,8-tetramethyl-2,3-dihydrobenzo[b][1,4] oxathiin-6-ol (64, TC393)** To a solution of **63** (0.22 mmol) in dry THF (4.4 mL), a solution of TBAF in dry THF (4.4 mL, 0.05M) was added. The reaction mixture was then stirred at ambient temperature for 40 minutes. Upon completion of the reaction, the mixture was diluted with CH₂Cl₂ and washed with sat. aq. NH₄Cl solution. The organic layer was washed with brine, dried over anhydrous Na₂SO₄ and evaporated in vacuo. Purification of the residue by flash-column chromatography (Hexane/Acetone 75:25, 7:3) afforded compound **64** as a yellow oil (yield over 2 steps starting from **62:** 27.4 mg, 27%). ¹H NMR (600 MHz, CDCl₃): δ 6.75 (d, *J* = 8.1 Hz, 1H, Ar), 6.69 (d, J = 1.8 Hz, 1H, Ar), 6.65 (dd, *J* = 8.1, 1.8 Hz, 1H, Ar), 4.41 (s, 1H, OH), 3.84 (2s, 6H, 2OCH₃), 3.51, 3.17 (AB_{q}, *J_{AB}* = 13.1 Hz, 2H, CH₂S), 3.13 (t,*J* = 7.6 Hz, 2H, CH₂-oxadiazole), 3.09 - 2.93 (m, 2H, CH₂Ar), 2.15 (3s, 9H, 3CH₃Ar), 1.83 (s, 3H, CH₃). ¹³C NMR (150 MHz, CDCl₃): δ 167.6, 166.9, 149.2, 148.0, 146.4, 141.9, 132.1, 125.0, 120.5, 120.4, 117.3, 115.0, 111.7, 111.5, 72.3, 56.1, 56.0, 33.8, 32.3, 27.7, 25.6, 12.4, 12.3. HRMS (ESI): Calcd C₂₄H₂₉N₂O₅S [M+H]⁺ 457.17189, found C₂₄H₂₉N₂O₅S 457.17933, Calcd C₂₄H₂₈N₂O₅SNa [M+Na]⁺ 479.17189, found C₂₄H₂₈N₂O₅SNa 479.16075, Calcd C₂₄H₂₇N₂O₅S [M-H]⁻ 455.17189, found C₂₄H₂₇N₂O₅S 455.16336

### Example 10

**4-(2-(5-(6-Hydroxy-2,5,7,8-tetramethyl-2,3-dihydrobenzo[b][1,4]oxathiin-2-yl)-1,3,4-oxadiazol-2-yl)ethyl)benzene-1,2-diol (65, TC394)** To an ice-cold solution of **64** (0.033 mmol, 15 mg) in dry CH₂Cl₂ (0.5 mL), BF₃S(CH₃)₂ (0.342 mmol, 36 µL) was added. The reaction mixture was stirred at ambient temperature for 24 h. Upon completion of the reaction, the solvent was evaporated under nitrogen flow. Compound **65** was obtained as a yellow oil (2 mg, 14%), after flash-column chromatography purification (DCM / MeOH 95:5) of the residue. ¹H NMR (600 MHz, CDCl₃): δ 6.66 (d, *J* = 8.0 Hz, 1H, Ar), 6.47 (s, 1H, Ar), 6.45 (d, *J* = 8.0 Hz, 1H, Ar), 3.52, 3.20 (AB_{q}, *J_{AB}* = 13.1 Hz, 2H, CH₂S), 3.09 (t, *J* = 7.3 Hz, 2H, CH₂-oxadiazole), 3.01 - 2.81 (m, 2H, CH₂Ar), 2.16 (s, 3H, CH₃), 2.13 - 2.12 (2s, 6H, 2CH₃), 1.84 (s, 3H, CH₃). ¹³C NMR (150 MHz, CDCl₃): δ 167.6, 167.0, 146.3, 143.8, 142.6, 142.0, 131.9, 125.1, 120.64, 120.59, 117.4, 115.5, 115.4, 114.9, 72.3, 33.7, 32.0, 27.6, 26.1, 12.4. HRMS (ESI): Calcd C₂₂H₂₃N₂O₅S [M-H]⁻ 427.14059, found C₂₂H₂₃N₂O₅S 427.13266

### EXPERIMENTAL PROCEDURES-RESULTS OF BIOLOGICAL ASSAYS

The following potetntial properties of the compounds under investigation were examined:
**1. Proteasome activation properties** were tested through detection of chymotrypsin-like proteasome activity following treatment of human primary fibroblast cultures with the compound under investigation (*in cellulo).* Any compound that induced proteasome activation (increased levels of chymotrypsin-like activity) was further investigated to reveal whether the activation occurs: (a) at the structural level through allosteric interaction of the compound with the complex, or, (b) at the transcriptional/translational level thus leading to quantitative increase of proteasome complexes. To distinguish between the two modes of proteasome activation, we studied either the activation of purified, isolated proteasome upon addition of the compound under investigation in the test tube (*in vitro*; scenario (a)) or the induction of protein expression levels of proteasome subunits (scenario b).
**2.** Their **anti-ageing properties** in a cellular system upon addition of each compound in human primary fibroblasts throughout their cellular lifespan were revealed through the measurement of the number of population doublings (PDLs) performed by the cultures. These PDLs we compared to PDLs performed by control cultures of human fibroblasts that were treated only with the diluent of the compound.
**3.** Their **anti-ageing properties** in a multi-cellular system upon addition of each compound in cultures of the nematode worm *Caenorhabditis elegans* (*C. elegans*) throughout their lifespan were revealed through the measurement of the median and maximum lifespan of the populations. These parameters we compared to the relative ones performed by control cultures of *C*. *elegans* that were treated only with the diluent of the compound.
**4.** Their **antioxidant properties** in cell cultures though the detection of the intracellular levels of carbonylated (oxidized) proteins (oxyblot analysis).

### PROTEASOME COMPLEX ACTIVATION AND ANTIAGEING PROPERTIES

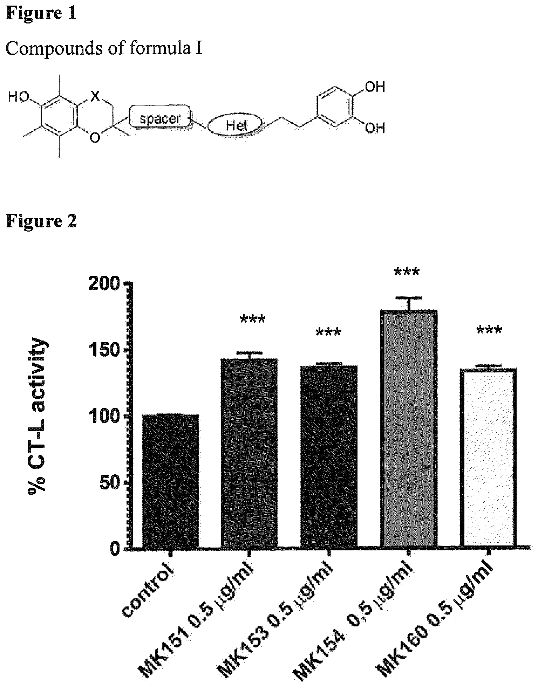

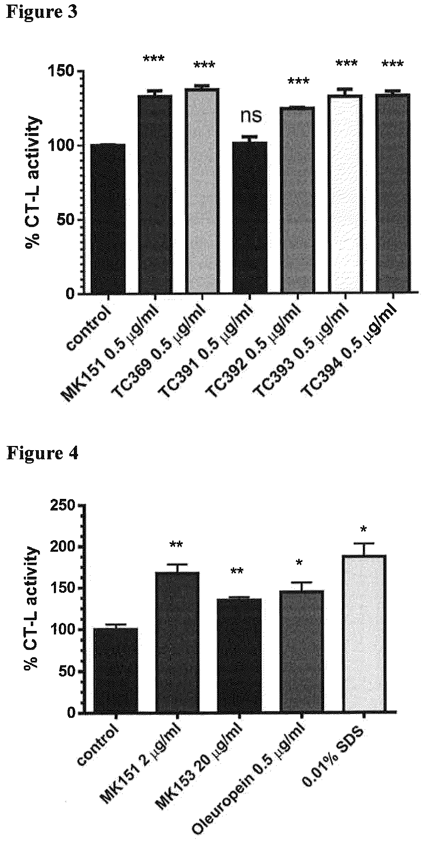

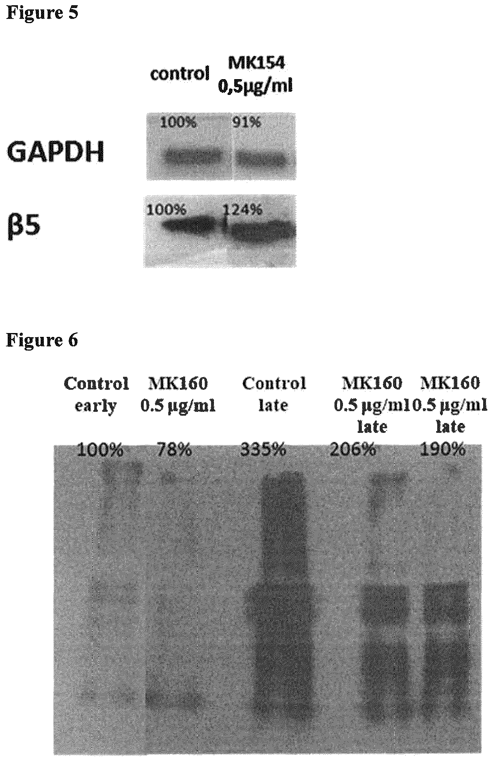

The steps, methods and results that were obtained regarding the proteasome-activating properties and the anti-ageing and anti-oxidation properties of the examined compounds are shown in Figures **2** and **3****:**
1) **Detection of chymotrypsin-like activity of the proteasome following incubation with the compound of formula I.**
**1.1.** HFL-1 human primary fibroblasts were cultured in complete medium [DMEM (Dulbecco's modified Eagle's medium: Invitrogen Life Technologies Inc.), supplemeted with 10% (v/v) bovine serum, 100 Units/ml penicillin, 100 µg/ml streptomycin, 2mM L-gutamine 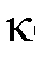 α 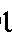 1% (v/v) non-essential aminoacids]. Following incubation of the cells with 4 different concentrations (0.5, 2, 5, 10 µg/ml) of various compounds of formula I or incubation with the appropriate diluent (0,1% DMSO) for 24 h, cells were collected, lysed and proteasome activities assays were performed in protein lysates. Detection of the main proteasome activity (chymotrypsin-like; degrading after hydrophobic residues) was performed in crude protein extracts with the hydrolysis of the fluorogenic peptide LLVY-AMC, for 30 min at 37 °C. Proteasome activity was determined as the difference between the total activity of crude extracts and the remaining activity in the presence of 10 µM MG132, a specific proteasome inhibitor. Fluorescence was measured using a VersaFluor^{™} fluorescence spectrophotometer (BioRad Laboratories Inc.). Protein concentrations were determined using the Bradford method with BSA as a standard.
**1.2.** **Figure 2** shows the percentage (%) of CT-L activity in HFL-1 human primary cells following treatment with the compounds under investigation for 24 h. Mean value of activity in DMSO-treated cells (control cultures) set to 100%. Error bars denote ± SEM. ***P-value<0.001. The chymotrypsin-like proteasome activity (CT-L) was induced by ~1.4-1.8 folds *in cellulo* in the presence of 0.5 µg/ml MK151, MK154 and MK169 as compared to the activity detected in the relative control cultures. **Figure 3** shows the percentage (%) of chymotrypsin-like (CT-L) activity in HFL-1 human primary cells following treatment with the compounds under investigation for 24 h. TC391 does not induce CT-L activity at this concentration. Mean value of activity in DMSO-treated cells (control cultures) set to 100%. Error bars denote ± SEM. ***P-value<0.001, ns: not significant. CT-L was induced by ~1.25-1.4 folds the presence of 0.5 µg/ml TC369, TC392, TC393 and TC394 as compared to the activity detected in the relative control cultures. CT-L activity was not induced by TC391 in the same concentration (0.5 µg/ml) but it was induced in a concentration of 5 µg/ml.
**1.3.** To reveal whether the observed proteasome activation is an activation mediated at the structural level through structural changes of the complex, 0.5 µg of purified, isolated proteasome were incubated in a test tube with compounds of formula I and the fluorogenic peptide LLVY-AMC, for 30 min at 37 °C. In a parallel assay, incubation was also performed in the presence of 10 µM of MG132 proteasome inhibitor. Fluorescence was measured using a VersaFluor^{™} fluorescence spectrophotometer (BioRad Laboratories Inc.). Samples that exhibited proteasome activation in the test tube reveal "structural" proteasome activation since the test tube is free of protein lysates, containing only purified proteasome and the incubation was performed in the absence of alive cells.
**1.4.** **Figure 4** shows the percentage (%) of chymotrypsin-like (CT-L) activity in isolated pure proteasome (in the absence of cell lysates) following incubation with the investigated compounds for 30 min. Oleuropein and SDS were used as positive controls. Only the best performing concentrations are shown. Mean value of activity in DMSO-treated proteasome (control sample) set to 100%. Error bars denote ± SEM. *P-value<0.05, **P-value<0.01. MK151 and MK153 induced CT-L activity by 1.3-1.7 folds through direct action on the purified, isolated proteasome.
**1.5.** To reveal whether the observed proteasome activation is mediated at the protein expression level leading to enhanced levels of proteasome complexes, HFL-1 human primary fibroblasts were cultured as described in 1.1. Following incubation with various compounds of formula I for 24 h, cells were collected, lysed and proteins were extracted and subjected to SDS-PAGE (sodium dodecylsulfate polyacrylamide gel electrophoresis) followed by immunoblot analysis against different proteasome subunits. Protein concentrations were determined using the Bradford method with BSA as a standard.
**1.6.** **Figure 5** shows the immunoblot analysis of β5 proteasome subunit in human primary fibroblasts treated with DMSO (control) or 0,5 µg/ml MK154. GAPDH was used as a loading control. Quantification of the ratio of each detected protein to GAPDH and normalization to control appears on the representative blot. The value exhibited by the control sample (cells treated with DMSO; Control early) was set as 100%. In the presence of MK154 elevated protein levels of β5 proteasome subunit were revealed, showing a quantitative enhancement of proteasome complexes. In total, the increased amount of proteasome subunits expression levels provide an explanation for the increased proteasome activities recorded in these cells.

**2) Study of the lifespan of human primary fibroblasts *in vitro* in the presence of the compounds of formula I.**
**2.1.** HFL-1 human primary fibroblasts were used in *in vitro* conditions in complete medium (growth at 37°C, 5% CO2 and 95% humidity). The cells were maintained constantly in culture in complete medium supplemented with compounds of formula I (with the final concentration ranging from 0,11 µM to 24,42 µM). Control cultures were supplmented with 0.1% DMSO. The cells were replenished with fresh media supplemented with the relative compounds or the diluents every 24 h and their numbers were measured using a Coulter Z2 counter (Coulter Corporation) until they reached senescence (approximately after 13 weeks). The cumulative population doublings performed by each culture were calculated using the following formula: CPD=Σ[PD] where PD=LOG[Nfinal/Ninitial]/LOG[2], where N final represents the number of cells that were measured when each culture reached confluence and N initial represents the number of cells that was initially seeded.
**2.2. Table 1** shows the total number of cumulative population doublings (CPDs) performed by the different HFL-1 cultures that were treated with the various compounds of formula I. More specifically, cells that were incubated with MK151 compound performed up to 12% more population doublings as compared to the population doublings performed by the relative control cultures. Likewise, cells that were incubated with MK154 and MK160 compounds performed 4% and 8% more population doublings, respectively, as compared to the population doublings performed by the relative control cultures. Consequently, MK151, MK154 and MK160 compounds possess anti-ageing properties that lead to cellular lifespan extension.

**Table 1: Number of CPDs of HFL-1 human primary fibroblasts treated with the compounds of formula I or the relative diluents for throughout their lifespan. Only the best performing concentrations are shown. Control cutlures were treated with the diluent (DMSO).**

| **Compound** | **CPDs (compound/diluent)** |
|---|---|
| **MK151** 0.5 µg/mL (1.22 µM) | 64.5/57.6 |
| **MK154** 0.05 µg/mL (0.11 µM) | 59.9/57.6 |
| **MK160** 0.25 µg/mL (0.61 µM) | 62.2/57.6 |

**3) Study of the organismal lifespan of the nematode worm *C*. *elegans* in the presence of the compounds of formula I.**
**3.1.** Wild type nematode worms *C.elegans* were maintained in NGM medium (Nematode Growth Medium) at 20°C throughout their lifespan. To reveal the median and maximum lifespan of each population, the procedure was as follows: Synchronized young adult animals were transferred to fresh plates (100-200 individuals/experiment) containing a specific concentration of each compound of formula I or the relative diluent (DMSO). Day 1 of adulthood was set as t=0. Animals were transferred to fresh plates every 2-3 days (to have access to fresh food/compound of formula I and to seperate the initial population from its descendants) and examined every day for touch-provoked movement and pharyngeal pumping until death. Each survival assay was repeated at least twice. Survival curves were created using the product-limit method by Kaplan and Meier. The percentage of nematodes remaining alive is plotted against animal age. The log-rank (Mantel-Cox) test was used to evaluate differences between survival curves and to determine *P* values for all independent data. Censored animals that died due to internally hatched eggs, extruded gonad or desiccation due to crawling off the plates were excluded. Median lifespan values are expressed as mean±SEM. Maximum lifespan of each population shows the age (in days) of the last animal remained alive in each culture.
**Table 2** shows median and maximum lifespan (with statistical significance) of wt *C*. *elegans* cultures following treatment with the mentioned compounds throughout the lifespan of the organisms compared to the relative control cultures. More specifically, cultures treated with MK151 and MK154 compounds exhibited a 8.2% higher median lifespan compared to the relative control cultures. Consequently, MK151 and MK154 compounds exhibit anti-ageing properties leading to organismal lifespan extension. It is of note that those two compounds had also led to cellular lifespan extension as shown in **Table 1.**

**Table 2: Median and maximum lifespan of wt C.elegans cultures following treatment with the mentioned compounds throughout the lifespan of the organisms. The concentrations in µg/ml refer to ml of food. The best concentrations are shown. Control cultures were treated with the diluent (DMSO).**

| **Compound** | **Mean lifespan (compound/diluent)** | **Max lifespan (compound/diluent)** |
|---|---|---|
| **MK151** 5 µg/mL (12.22 µM) | **22** ± 0.57 / 20.33 ± 0.33 | **32**/28 |
| **MK154** 2.5 µg/mL (5.5 µM) | **22** / 20.33 ± 0.33 | **32**/28 |

**4) Detection of intracellular levels of oxidized proteins (Oxyblot analysis) in the presence of the compounds of formula I.**
**4.1.** HFL-1 HDFs were cultured as described in 1.1. Cells treated with the compounds for 24 h and were then lysed and proteins were extracted and subjected to SDS-PAGE followed by OxyBlot analysis that detects the carbonylated groups on proteins that arises during protein oxidation. OxyBlot^{™} is a protein oxidation detection kit.
**4.2.** **Figure 6** shows the oxyblot analysis of protein cell lysates following constant treatment of cells with the investigated compounds throughout their lifespan starting from early passage (early) until the end of their proliferative capacity (late). Only the best performing concentrations are shown. Quantification of the ratio of each detected protein to GAPDH and normalization to control appears on the representative blot as percentages. The value exhibited by the early passage control treated with DMSO (Control early) was set as 100%. Cells treated with MK151, MK154 and MK160 had less oxidized proteins as compared to the relative control cells. Consequently, these compounds possess anti-oxidant activity.

## Claims

1. A hybrid compound of formula I 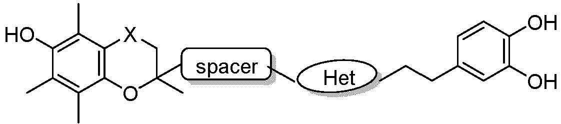 wherein
X = CH₂ or S
Spacer = (CH₂)ₙ, n=0 or CH₂OCH₂
Het is selected from

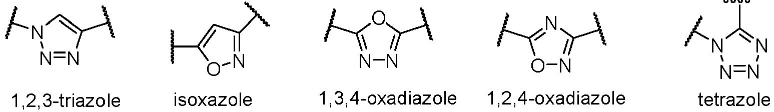

2. The compound according to claim 1, selected from the following compounds

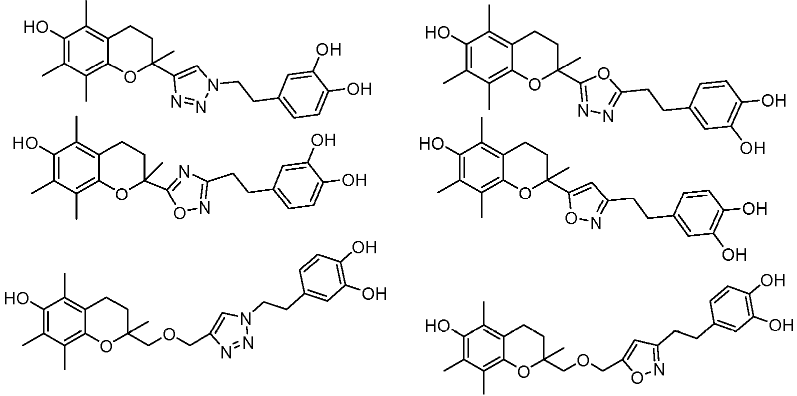

3. The compound according to claim 1 selected from the following compounds

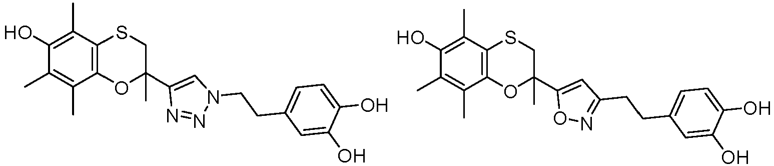

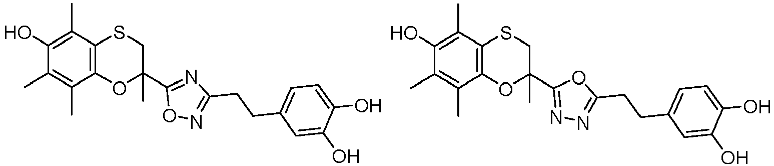

4. The compound of formula I as defined in any one of claims 1-3 for use in the prevention and lessening of the symptoms of age-related diseases associated with reduced activity and function of proteasome, namely Alzheimer and Huntington diseases.

5. A composition comprising the compound of formula I as defined in any one of claims 1-3.

6. In vitro use of a compound of formula I as defined in any one of claims 1-3 as a control compound of proteasome activation in an anti-aging product.

7. Cosmetic non-therapeutic use of a compound of formula I as defined in any one of claims 1-3 as topical or transdermal anti-ageing component in a composition.

## Patentansprüche

1. Hybridverbindung der Formel I 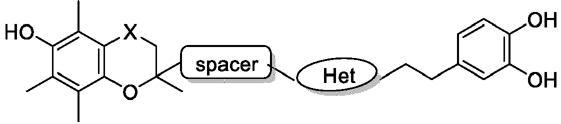 wobei
X = CH₂ oder S
Spacer = (CH₂)ₙ , n=0 oder CH₂OCH₂
Het ist ausgewählt aus

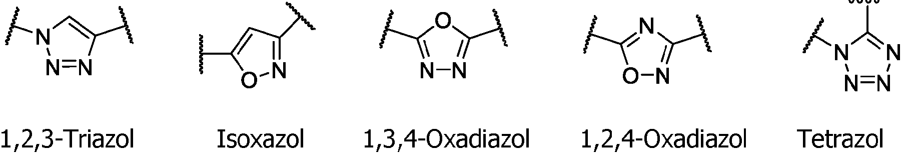

2. Verbindung nach Anspruch 1, ausgewählt aus den folgenden Verbindungen

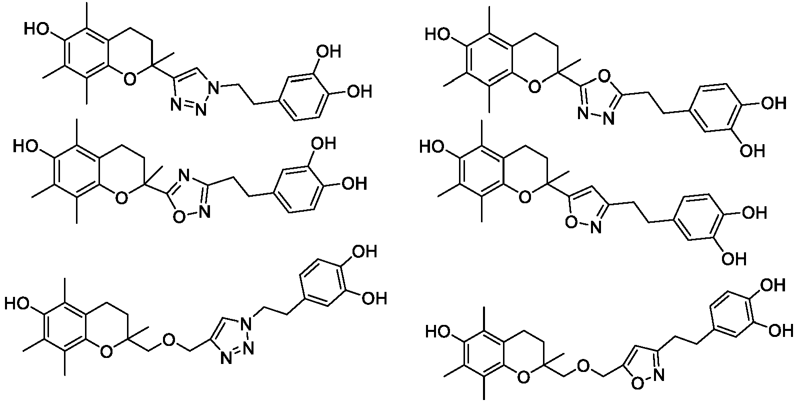

3. Verbindung nach Anspruch 1, ausgewählt aus den folgenden Verbindungen

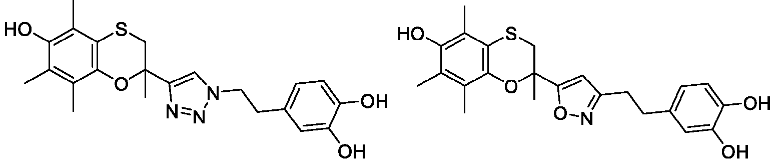

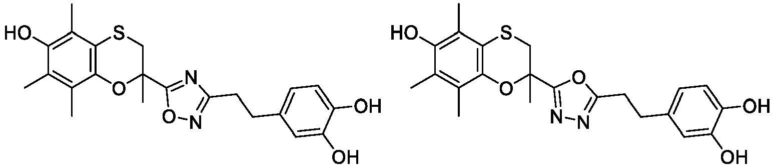

4. Verbindung der Formel I, wie in einem der Ansprüche 1-3 definiert, zur Verwendung bei der Vorbeugung und Linderung der Symptome von altersbedingten Krankheiten, die mit einer verminderten Aktivität und Funktion des Proteasoms verbunden sind, nämlich Alzheimer- und Huntington-Krankheit .

5. Zusammensetzung, umfassend die Verbindung der Formel I, wie in einem der Ansprüche 1 bis 3 definiert.

6. In vitro-Verwendung einer Verbindung der Formel I, wie in einem der Ansprüche 1 bis 3 definiert, als Kontrollverbindung für die Proteasom-Aktivierung in einem Anti-Aging-Produkt.

7. Kosmetische nicht-therapeutische Verwendung einer Verbindung der Formel I, wie in einem der Ansprüche 1 bis 3 definiert, als topische oder transdermale Anti-Aging-Komponente in einer Zusammensetzung.

## Revendications

1. Composé hybride de formule I 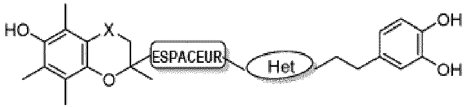 dans laquelle
X = CH₂ ou S
Espaceur = (CH₂)ₙ, n=0 ou CH₂OCH₂
Het est choisi parmi

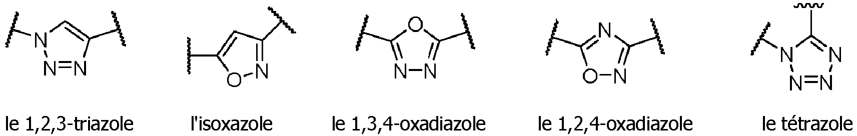

2. Composé selon la revendication 1, choisi parmi les composés suivants

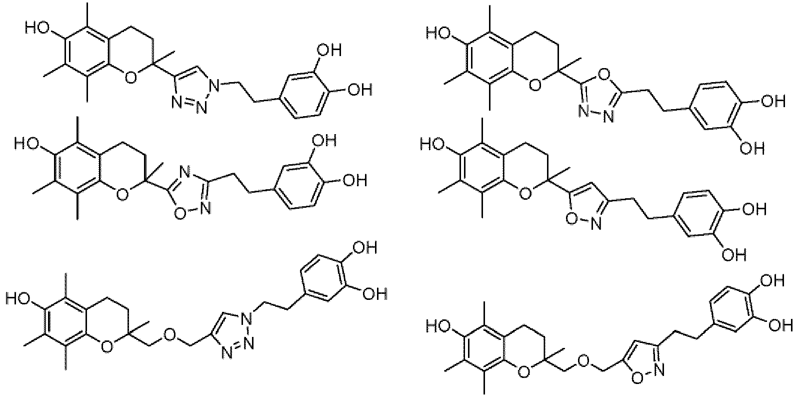

3. Composé selon la revendication 1, choisi parmi les composés suivants

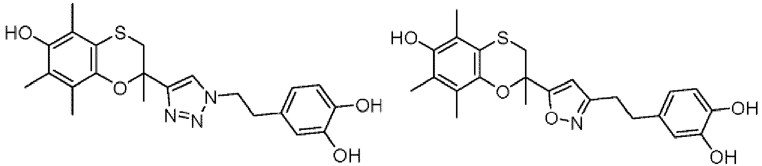

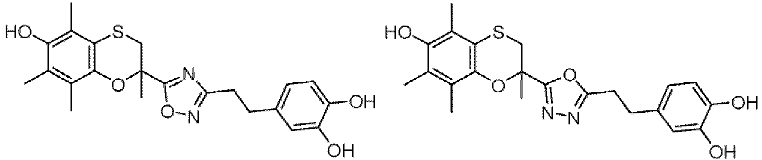

4. Composé de formule I tel que défini dans l'une quelconque des revendications 1 à 3 pour une utilisation dans la prévention et la réduction des symptômes de maladies liées à l'âge, associées à une activité et une fonction réduites du protéasome, à savoir les maladies d'Alzheimer et de Huntington.

5. Composition comprenant le composé de formule I tel que défini dans l'une quelconque des revendications 1 à 3.

6. Utilisation in vitro d'un composé de formule I tel que défini dans l'une quelconque des revendications 1 à 3 comme composé de contrôle de l'activation du protéasome dans un produit anti-âge.

7. Utilisation cosmétique non thérapeutique d'un composé de formule I tel que défini dans l'une quelconque des revendications 1 à 3 comme composant anti-âge topique ou transdermique dans une composition.
